# EUROPÄISCHE PATENTSCHRIFT

(11) **EP 3 931 231 B1**
(45) Veröffentlichungstag und Bekanntmachung des Hinweises auf die Patenterteilung: **25.09.2024**
(21) Anmeldenummer: 20705206.9
(22) Anmeldetag: 18.02.2020
(51) Int. Cl.: C08G 18/08, C08G 18/12, C08G 18/28, C08G 18/32, C08G 18/38, C08G 18/40, C08G 18/44, C08G 18/48, C08G 18/73, C08G 18/75, C08G 18/79, A61L 15/42, C08G 101/00, A61L 15/26

(54) **THERMOPLASTISCHER SCHAUM HERGESTELLT AUS ZWEI SPEZIELLEN POLYURETHANDISPERSIONEN**
THERMOPLASTIC FOAM MADE FROM TWO SPECIFIC POLYURETHANE DISPERSIONS
MOUSSE THERMOPLASTIQUE FABRIQUÉE À PARTIR DE DEUX DISPERSIONS DE POLYURÉTHANE SPÉCIALES

(30) Priorität: 28.02.2019 EP 19159939
(43) Veröffentlichungstag der Anmeldung: 05.01.2022
(73) Patentinhaber: Covestro Deutschland AG, 51373 Leverkusen (DE)
(72) Erfinder: WEISER, Marc-Stephan, 51515 Kürten-Dürscheid (DE); PLUG, Sascha, 51375 Leverkusen (DE); DÖRR, Sebastian, 40593 Düsseldorf (DE)
(74) Vertreter: Levpat
(86) Internationale Anmeldenummer: PCT/EP2020/054228
(87) Internationale Veröffentlichungsnummer: WO 2020/173760

(56) Entgegenhaltungen:
- US-A1- 2011 275 728

## Beschreibung

Die Erfindung betrifft einen geschäumten Artikel, insbesondere einen Hygieneartikel, ein Bestandteil einer Wundauflage, einen Bestandteil eines Wearable Devices oder einen Wundschaum, der auf Basis zweier Polyurethan-Dispersionen (A) und (B) erhältlich ist, sowie dessen Herstellungsverfahren, dessen Anwendung und der Bereitstellung eines Kit of Parts aus den beiden Polyurethan-Dispersionen (A) und (B).

Aus dem Stand der Technik sind geschäumte Artikel bekannt, die zur Bereitstellung eines stabilen Schaumes diverse Schaumstabilisatoren benötigen. Zumeist sind die verwendeten Schaumstabilisatoren nicht geeignet, um den Schaum in Kontakt mit der menschlichen Haut zu bringen, oder um daraus Wundauflagen herzustellen. Dies liegt insbesondere an dem zytotoxischen Potenzial der eingesetzten Additive in den verwendeten Formulierungen. Solche Schäume sind bekannt aus den Patentanmeldungen US-2016-0235880-A1 oder US-2011-0275728-A1.

Eine weitere Anforderung an geschäumte Artikel ist häufig, dass sie möglichst rissfrei sein sollen. Dies ist wiederum insbesondere wichtig, wenn diese geschäumten Artikel als Wundauflage Verwendung finden sollen. Die Risse weisen den Nachteil auf, dass sie sowohl die Stabilität des geschäumten Artikels insgesamt beeinträchtigen, aber auch Einfallstellen für Verunreinigungen sind und zumeist auf eine gewisse Sprödheit des Schaumes hinweisen sowie optisch von Kunden nicht akzeptiert werden.

Daher stellte sich als eine Aufgabe der anhängigen Erfindung einen geschäumten Artikel bzw. ein Herstellungsverfahren für einen solchen Artikel bereitzustellen, der mindestens einen Nachteil des Standes der Technik zumindest zum Teil nicht aufweist.

Eine weitere Aufgabe der Erfindung war es, einen geschäumten Artikel bereitzustellen, der bei hoher Hautverträglichkeit und möglichst geringer Menge an toxischen oder zytotoxischen Inhaltsstoffen eine hohe Stabilität und hohe Reißfestigkeit aufweist.

Darüber hinaus stellte sich die Aufgabe, ein Verfahren bereitzustellen, das die Herstellung eines möglichst stabilen, reißfesten und/oder gut hautverträglichen, insbesondere nicht-toxischen oder nicht-zytotoxisch geschäumten Artikels ermöglicht.

Ein erster Gegenstand der anhängigen Erfindung ist ein geschäumter Artikel, hergestellt durch Mischen einer ersten Polyurethan-Dispersion (A) mit wenigstens einer zweiten Polyurethan-Dispersion (B), gegebenenfalls unter Zusatz weiterer Additive, Aufschäumen und anschließendem Trocknen der Mischung, wobei die Polyurethan-Dispersion (A) erhältlich ist, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol, oder bevorzugt von 600 bis 3000 g/mol, und OH-Funktionalitäten von 1,5 bis 6, bevorzugt 1,8 bis 3, oder bevorzugt von 1,9 bis 2,1, und
   A3) gegebenenfalls hydroxyfunktionellen Verbindungen mit Molekulargewichten von 62 bis 399 g/mol, und
   A4) gegebenenfalls isocyanatreaktiven, anionischen oder potentiell anionischen und gegebenenfalls nichtionischen Hydrophilierungsmitteln,
      hergestellt, und
B) deren freie NCO-Gruppen dann ganz oder teilweise
   B1) gegebenenfalls mit aminofunktionellen Verbindungen mit Molekulargewichten von 32 bis 400 g/mol und
   B2) mit aminofunktionellen, anionischen oder potentiell anionischen Hydrophilierungsmitteln
      unter Kettenverlängerung umgesetzt werden und die Prepolymere vor, während oder nach Schritt B) in Wasser dispergiert werden, wobei gegebenenfalls enthaltene potentiell anionische Gruppen durch teilweise oder vollständige Umsetzung mit einem Neutralisationsmittel in die anionische Form überführt werden, und
   wobei die zweite Polyurethan-Dispersion (B) erhältlich ist durch reaktive Umsetzung wenigstens folgender Komponenten:

A. einer aliphatischen Polyisocyanat-Komponente mit einer mittleren Isocyanatfunktionalität von ≥ 1,8 und ≤ 2,6,
B. einer polymeren Polyether-Polyol-Komponente,
C. einer aminofunktionellen Kettenverlängerer-Komponente mit mindestens 2 isocyanatreaktiven Aminogruppen, enthaltend wenigstens eine aminofunktionelle Verbindung C 1., die keine ionischen oder ionogenen Gruppen aufweist und/oder eine aminofunktionelle Verbindung C2., die ionische oder ionogene Gruppen aufweist,
D. gegebenenfalls weiteren hydrophilierenden Komponenten, die unterschiedlich sind von C2.,
E. gegebenenfalls hydroxyfunktionellen Verbindungen mit einem Molekulargewicht von 62 bis 399 mol/g,
F. gegebenenfalls weiteren polymeren Polyolen, welche unterschiedlich sind von B.,
G. einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
H. optional einer aliphatischen Polyisocyanat-Komponente mit einer mittleren Isocyanatfunktionalität von > 2,6 und ≤ 4,
wobei die Komponenten B. und F. zusammen ≤ 30 Gew.-% an Komponente F., bevorzugt ≤ 20 Gew.-% an Komponente F., oder bevorzugt ≤ 10 Gew.-% an Komponente F., bezogen auf die Gesamtmasse der Komponenten B. und F. enthalten.

Die Polyurethan-Dispersionen (A) und (B) unterscheiden sich in ihrer Zusammensetzung mindestens in der Auswahl oder Menge mindestens einer Komponente. Polyurethan-Dispersionen (A) und (B) sind folglich mindestens in einem Teil des jeweiligen Polymers chemisch verschieden und voneinander unterscheidbar.

Bevorzugt sind die Bestandteile der Dispersionen (A) und (B) sowie alle weiteren Komponenten zur Bildung des geschäumten Artikels gering bis nicht-zytotoxisch. Unter gering bis nicht-zytotoxisch gemäß der Erfindung wird verstanden, dass der geschäumte Artikel und bevorzugt sämtliche Bestandteile des geschäumten Artikels eine Viabilität von ≥ 70 %, bevorzugt ≥ 80 %, beziehungsweise eine Klassifizierung von 0 bis 2 ("no to mild", was keine bis geringe Zytotoxizität bedeutet) in einem Zytotoxtest nach DIN ISO 10993-5:2009-10 aufweisen.

### Polyurethan-Dispersion (A)

Um bei der Herstellung der Polyurethan-Dispersion (A) eine anionische Hydrophilierung zu erreichen, werden in A4) und/oder B2) bevorzugt Hydrophilierungsmittel eingesetzt, die wenigstens eine gegenüber NCO-Gruppen reaktive Gruppe wie im Falle von A4) Amino-, Hydroxy- oder Thiolgruppen und im Falle von B2) Aminogruppen aufweisen und darüber hinaus -COO- oder -SO₃- oder -PO₃²⁻ als anionische bzw. deren ganz oder teilweise protonierte Säureformen als potentiell anionische Gruppen aufweisen.

Bevorzugte wässrige, anionische Polyurethan-Dispersionen (A) haben einen niedrigen Grad an hydrophilen anionischen Gruppen, bevorzugt von 2 bis 200 Milliäquivalenten, besonders bevorzugt von weniger als 10 bis 100 Milliäquivalenten pro 100 g Festharz.

Um eine gute Sedimentationsstabilität zu erreichen, liegt die zahlenmittlere Teilchengröße der Polyurethan-Dispersionen (A) bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5; bevorzugt 1,2 bis 3,0 und besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt 50 bis 125 %, besonders bevorzugt 60 bis 100 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4`-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C1-C8-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mn von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester, wobei 1,6-Hexandiol und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden. Die Hydroxylgruppen aufweisenden Polycarbonate sind bevorzugt linear gebaut.

Ebenfalls können in A2) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (A) enthalten als Komponente A2) eine Mischung aus Polycarbonatpolyolen und Polytetramethylenglykolpolyolen, wobei in dieser Mischung der Anteil an Polycarbonatpolyolen in der Mischung 0 bis 80 Gew.-% und der Anteil an Polytetramethylenglykolpolyolen 100 bis 20 Gew.-% beträgt. Bevorzugt ist ein Anteil von 50 bis 100 Gew.-% an Polytetramethylenglykolpolyolen und ein Anteil von 0 bis 50 Gew.-% an Polycarbonatpolyolen. Besonders bevorzugt ist ein Anteil von 75 bis 100 Gew.-% an Polytetramethylenglykolpolyolen und ein Anteil von 0 bis 25 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.-%, bevorzugt 60 Gew.-% und besonders bevorzugt mindestens 70 Gew.-% beträgt.

Die Verbindungen der Komponente A3) besitzen Molekulargewichte von 62 bis 400 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxycapronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können in A3) auch monofunktionelle, isocyanatreaktive, hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5-9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw. Carbonsäuregruppen als ionische oder potentiell ionische Gruppen enthalten, wie Dimethylolpropionsäure, Dimethylolbuttersäure und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol-% Ethylenoxid- und 0 bis 60 mol-% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclo-hexylaminopropan, 3-Amino-1-Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin. Besonders bevorzugt werden Mischungen der vorgenannten Diamine der Komponente B1) eingesetzt, insbesondere Mischungen aus 1,2-Ethylendiamin und Isophorondiamin sowie Mischungen aus 1,4-Diaminobutan und Isophorondiamin.

Unter anionisch bzw. potentiell anionisch hydrophilierenden Verbindungen der Komponente B2) werden sämtliche Verbindungen verstanden, die mindestens eine Amino-Gruppe aufweisen, sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiamin-propyl- oder - butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen (A) werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0 bis 20 Gew.-% Summe der Komponenten A3) und B1)
0,1 bis 25 Gew.-% Summe der Komponenten A4) und B2), wobei bezogen auf die Gesamtmengen der Komponenten A1) bis A4) und B1) bis B2) 0,1 bis 10 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen (A) werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,5 bis 15 Gew.-% Summe der Komponenten A4) und B2), wobei bezogen auf die Gesamtmengen der Komponenten A1) bis A4) und B1) bis B2) 0,5 bis 7 Gew.-% an anionischen bzw. potentiell anionischen Hydrophilierungsmitteln aus A4) und/oder B2) verwendet werden.

Die Herstellung der anionisch hydrophilierten Polyurethan-Dispersionen (A) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den genannten aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen 1,05 bis 3,5; bevorzugt 1,2 bis 3,0 und besonders bevorzugt 1,3 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (A) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (A) beträgt typischerweise weniger als 9,0, bevorzugt weniger als 8,5; besonders bevorzugt weniger als 8,0 und liegt ganz besonders bevorzugt bei 6,0 bis 7,5.

Der Feststoffgehalt der Polyurethan-Dispersionen (A) beträgt bevorzugt 35 bis 70 Gew.-%, besonders bevorzugt 40 bis 65 Gew.-%, ganz besonders bevorzugt 45 bis 60 Gew.-% und insbesondere 45 bis 55 Gew.-%.

Die Menge an anionischen oder potentiell anionischen Gruppen auf der Partikeloberfläche, gemessen über eine Säure-Base-Titration liegt im Allgemeinen bei 2 bis 500 mmol, bevorzugt 30 bis 400 mmol pro 100 Gramm Festkörper.

### Polyurethan-Dispersion (B)

Bei der Herstellung der Polyurethan-Dispersion (B) wird zunächst ein isocyanatgruppenhaltiges Prepolymer gebildet, das mit Aminogruppen Kettenverlängert wird. Aufgrund der Verwendung von Aminogruppen-haltigen Verbindungen bei der Herstellung der Polyurethan-Dispersion (B) können auch Harnstoffgruppen entstehen, warum im Folgenden das Polyurethan, das in der Polyurethan-Dispersion (B) enthalten ist auch Polyurethanharnstoff genannt wird und daher die Polyurethan-Dispersion (B) auch als Polyurethanharnstoff-Dispersion (B) bezeichnet wird.

Unter ionogenen Gruppen werden im Zusammenhang mit der Polyurethanharnstoff-Dispersion (B) solche funktionellen Gruppen verstanden, die in der Lage sind, beispielsweise durch Neutralisation mit einer Base, ionische Gruppen zu bilden.

Die Komponente A. kann jedes Polyisocyanat sein, das der Fachmann hierfür verwenden würde. Als Komponente A. bevorzugt geeignete Polyisocyanate sind insbesondere die dem Fachmann an sich bekannten aliphatischen Polyisocyanate mit einer mittleren Isocyanat-funktionalität von ≥ 1,8 und ≤ 2,6. Der Begriff aliphatisch umfasst dabei auch cycloaliphatische und/oder araliphatische Polyisocyanate.

Unter der mittleren Isocyanatfunktionalität wird dabei die durchschnittliche Anzahl an Isocyanatgruppen pro Molekül verstanden.

Bevorzugte Polyisocyanate sind solche des Molekulargewichtsbereichs von 140 bis 336 g/mol. Besonders bevorzugt sind diese ausgewählt aus der Gruppe bestehend aus. 1,4-Diisocyanatobutan (BDI), 1,5-Pentandiisocyanat, (PDI) 1,6-Diisocyanatohexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1-methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatononan (TIN)), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan sowie die cycloaliphatischen Diisocyanate 1,3- bzw. 1,4-Diisocyanatocyclohexan, 1,4-Di¬isocyanato-3,3,5-trimethylcyclohexan, 1,3-Diisocyanato-2(4)-methylcyclohexan, 1-Isocyanato-3,3,5-trimethyl-5-isocyanatomethyl-cyclohexan (Isophorondiisocyanat, IPDI), 1-Isocyanato-1-methyl-4(3)isocyanato-me-thylcyclohexan, 1,8-Diisocyanato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4' und/oder 2,4'-Diisocyanatodicyclohexylmethan, 4,4' Diisocyanato-3,3'-dimethyldicyclohexylmethan, 4,4' Diisocyanato-3,3',5,5'-tetramethyldicyclohexylmethan, 1,3-Diisocyanatoadamantan, und 1,3-Dimethyl-5,7-diisocyanatoadamantan oder beliebige Gemische solcher Isocyanate. Ganz besonders bevorzugt sind die Polyisocyanate aus-gewählt aus 1,4-Butylendiisocyanat, 1,5-Pentamethylendiisocyanat (PDI), 1,6-Hexamethy-lendiisocyanat (HDI), Isophorondiisocyanat (IPDI), 2,2,4- und/oder 2,4,4-Trimethylhexamethylendiisocyanat, die isomeren Bis-(4,4`-isocyanatocyclohexyl)methane oder deren Mischungen beliebigen Isomerengehalts (H12-MDI), 1,4-Cyclohexylendiisocyanat, 4-Isocyanatomethyl-1,8-octandiisocyanat (Nonantriisocyanat) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanu-rat-, Urethan-, Allophanat-, Biuret-, Iminooxadiazindion- oder Oxadiazintrionstruktur sowie Mischungen aus diesen und/oder den oben stehenden anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocya-natgruppen oder Mischungen aus diesen und einer mittleren NCO-Funktionalität der Mi-schung von ≥ 1,8 und ≤ 2,6 und besonders bevorzugt ≥ 2,0 und ≤ 2,4.

Besonders bevorzugt enthält die organische Polyisocyanat-Komponente A. ein aliphatisches oder cycloaliphatisches Polyisocyanat ausgewählt aus HDI, IPDI und/oder H12-MDI oder deren Modifikationsprodukten, ganz besonders bevorzugt ausgewählt aus HDI und/oder IPDI.

In einer bevorzugten Variante liegen als Komponente A. IPDI und HDI im Gemisch vor.

Das Gewichtsverhältnis von IPDI:HDI liegt dabei bevorzugt im Bereich von 1,05 bis 10, besonders bevorzugt im Bereich von 1,1 bis 5, und ganz besonders bevorzugt im Bereich von 1,1 bis 1,5.

Bevorzugt wird zur Herstellung des erfindungsgemäß verwendeten Polyurethanharnstoffs ≥ 5 und ≤ 40 Gew.-% der Komponente A. und besonders bevorzugt ≥ 10 und ≤ 35 Gew.-% der Komponente A., jeweils bezogen auf die Gesamtmasse des Polyurethanharnstoffs, eingesetzt.

Bevorzug wird zur Herstellung des Polyurethanharnstoffs auch die Komponente H., eine aliphatische Polyisocyanat-Komponente mit einer mittleren Isocyanatfunktionalität (mittlere Anzahl Isocyanatgruppen pro Molekül) von > 2,6 und ≤ 4, vorzugsweise ≥ 2,8 und ≤ 3,8 eingesetzt. Die Komponente H. wird dabei bevorzugt im Gemisch mit Komponente A. eingesetzt.

Als Komponente H. besonders geeignet sind oligomere Diisocyanate, die eine Funktionalität > 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält H. Isocyanuratstrukturen.

Bevorzugt besteht die organische Polyisocyanat-Komponente H. aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente A. zu Komponente H. beträgt dabei bevorzugt 100:0,5 bis 100: 50; besonders bevorzugt 100: 2 bis 100: 15 und ganz besonders bevorzugt 100:3 bis 100: 8.

Bevorzug wird zur Herstellung des erfindungsgemäß verwendeten Polyurethanharnstoffs ≥ 0 und ≤ 10 Gew.-% der Komponente H. und besonders bevorzugt ≥ 0,1 und ≤ 3 Gew.-% der Komponente H., jeweils bezogen auf die Gesamtmasse des Polyurethanharnstoffs, eingesetzt.

Die erfindungsgemäß als Komponente B. eingesetzten polymeren Polyether-Polyole weisen bevorzugt zahlenmittlere Molekulargewichte von ≥ 500 und ≤ 8000 g/mol, bestimmt durch Gelpermeationschromatographie gegenüber Polystyrol-Standard in Tetrahydrofuran bei 23°C, bevorzugter ≥ 400 und ≤ 6000 g/mol und besonders bevorzugt von ≥ 600 und ≤ 3000 g/mol und/oder OH-Funktionalitäten von bevorzugt ≥ 1,5 und ≤ 6, bevorzugter ≥ 1,8 und ≤ 3, besonders bevorzugt von ≥ 1,9 und ≤ 2,1 auf. Der Ausdruck "polymere" Polyether-Polyole bedeutet hier insbesondere, dass die genannten Polyole mindestens drei, bevorzugter mindestens vier miteinander verbundene Wiederholungseinheiten aufweisen.

Das zahlenmittlere Molekulargewicht wird im Rahmen dieser Anmeldung bestimmt durch Gelpermeationschromatographie (GPC) in Tetrahydrofuran bei 23°C, außer wenn dies anders beschrieben wird. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzug enthält die Komponente B. Poly(tetramethylenglykol)polyetherpolyole oder besteht daraus.

Geeignete Poly(tetramethylenglykol)polyetherpolyole sind beispielsweise durch Polymerisation von Tetrahydrofuran mittels kationischer Ringöffnung erhältlich sind.

Bevorzugt enthält die Komponente B. ein Gemisch aus Poly(tetramethylenglykol)polyetherpolyolen oder besteht daraus, wobei sich die Poly(tetramethylenglykol)polyetherpolyole in ihren zahlenmittleren Molekulargewichten unterscheiden.

Erfindungsgemäß wird zur Herstellung des Polyurethanharnstoffs eine aminofunktionelle Kettenverlängerer-Komponente C. mit mindestens 2 isocyanatreaktiven Aminogruppen, enthaltend wenigstens eine aminofunktionelle Verbindung C 1., die keine ionischen oder ionogenen Gruppen aufweist und/oder eine aminofunktionelle Verbindung C2., die ionische oder ionogene Gruppen aufweist, eingesetzt.

Die aminofunktionellen Verbindungen der Komponente C. Komponente werden bevorzugt aus primären und/oder sekundären Diaminen ausgewählt. Insbesondere umfassen die aminofunktionellen Verbindungen C. mindestens ein Diamin.

Bevorzugt umfasst die aminofunktionelle Komponente C. wenigstens eine aminofunktionelle Verbindung C2., die ionische und/oder ionogene Gruppen aufweist.

Bevorzugt umfasst die aminofunktionelle Komponente C. sowohl aminofunktionelle Verbindungen C2., die ionische und/oder ionogene Gruppe aufweisen, als auch aminofunktionelle Verbindungen C1., die keine ionische oder ionogene Gruppe aufweisen.

Beispielsweise können als Komponente C1. organische Di- oder Polyamine wie beispielsweise 1,2-Ethylendiamin, 1,2- und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.

Bevorzugt ist die Komponente C1. ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.

Bevorzugt enthält die Komponente C1. ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1. bevorzugt in den genannten Bereichen vorliegt. Bevorzugt enthält die Komponente C1. ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin.

Bevorzugt umfasst die hydrophilierende Komponente C2. mindestens eine anionisch hydrophilierende Verbindung. Weiterhin bevorzugt beinhaltet die hydrophilierende Komponente C2. eine anionisch hydrophilierende Verbindung zu mindestens 80 Gew.-%, oder bevorzugt zu mindestens 90 Gew.-%, bezogen auf das Gesamtgewicht der Komponente C2.. Besonders bevorzugt besteht die Komponente C2. aus ausschließlich anionisch hydrophilierenden Verbindungen.

Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2. eine anionisch hydrophilierende Verbindung, die wenigstens eine anionische oder ionogene Gruppe und wenigstens zwei Aminogruppen aufweist oder besteht daraus.

Geeignete anionisch hydrophilierende Verbindungen als Komponente C2., im Weiteren auch Hydrophilierungsmittel C2. genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2. sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.

Bevorzugte anionische Hydrophilierungsmittel C2. sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure. Ganz besonders bevorzugt wird 2-(2-Aminoethylamino)ethylsulfonsäure oder deren Salze als anionisches Hydrophilierungsmittel C2. eingesetzt.

Gegebenenfalls kann die anionische Gruppe in der Komponente C2. auch eine Carboxylat- bzw. Carbonsäuregruppe sein. Die Komponente C2. wird dann bevorzugt aus Diaminocarbonsäuren ausgewählt. Bei dieser alternativen Ausführungsform müssen jedoch die Carbonsäure-basierenden Komponenten C2. in höheren Konzentrationen eingesetzt werden, verglichen mit solchen Komponenten C2., die Sulfonat- oder Sulfonsäuregruppen tragen. Besonders bevorzugt werden daher zur Herstellung des Polyurethanharnstoffs keine hydrophilierenden Verbindungen, die ausschließlich Carboxylatgruppen als anionische Gruppen der Komponente C2. tragen eingesetzt.

Bevorzugt wird zur Herstellung des erfindungsgemäß verwendeten Polyurethanharnstoffs in einem Bereich von ≥ 0,1 und ≤ 10 Gew.-% der Komponente C2. und besonders bevorzugt in einem Bereich von ≥ 0,5 und ≤ 4 Gew.-% der Komponente C2., jeweils bezogen auf die Gesamtmasse des Polyurethanharnstoffs, eingesetzt.

Zur Hydrophilierung können auch Mischungen aus anionischen Hydrophilierungsmitteln C2. und weiteren Hydrophilierungsmitteln D., welche von C2. verschieden sind, verwendet werden.

Geeignete weitere Hydrophilierungsmittel D. sind beispielsweise nichtionische hydrophilierende Verbindungen D1. und/oder hydroxyfunktionelle ionische oder ionogene, bevorzugt anionische oder anionogene, Hydrophilierungsmittel D2. Bevorzugt handelt es sich bei der Komponente D. um nichtionisch hydrophilierende Komponenten D1..

Geeignete hydroxyfunktionelle ionische oder ionogene Hydrophilierungsmittel als Komponente D2. sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionische oder ionogenen Hydrophilierungsmittel D2., insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2. in einem Bereich von 0 bis 1 Gew.-%, oder bevorzugt in einem Bereich von 0 bis 0,5 Gew.-%, bezogen auf die Gesamtmasse des Polyurethanharnstoffs in dem Polyurethanharnstoff enthalten.

Geeignete nichtionisch hydrophilierende Verbindungen als Komponente D1. sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bevorzugt enthält der verwendete Polyurethanharnstoff zur Bildung der Polyurethanharnstoff Dispersion (B) in einem Bereich von ≥ 0 und ≤ 20 Gew.-% der Komponente D., bevorzugt in einem Bereich von ≥ 0 und ≤ 10 Gew.-% der Komponente D. und ganz besonders bevorzugt in einem Bereich von ≥ 0 und ≤ 5 Gew.-% der Komponente D., jeweils bezogen auf die Gesamtmasse des Polyurethanharnstoffs. In einer weiteren bevorzugten Ausführungsform wird Komponente D. zur Herstellung des Polyurethanharnstoffs nicht verwendet.

Als Komponente E. können wahlweise Polyole, insbesondere nicht-polymere Polyole, des genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Bevorzugt enthält der verwendete Polyurethanharnstoff zur Bildung der Polyurethanharnstoff Dispersion (B) ≤ 10 Gew.-% der Komponente E., bevorzugt ≤ 5 Gew.-%, besonders bevorzugt 0 Gew.-% der Komponente E., jeweils bezogen auf die Gesamtmasse des Polyurethanharnstoffs. Bevorzugt beinhaltet der Polyurethanharnstoff die Komponente E. in einem Bereich von 0,1 bis 10 Gew.-%, bevorzugt in einem Bereich von 0,2 bis 8 Gew.-%, bevorzugt in einem Bereich von 0,1 bis 5 Gew.-%, bezogen jeweils auf die Gesamtmasse des Polyurethanharnstoffs. In einer weiteren bevorzugten Ausführungsform wird Komponente E. zur Herstellung des Polyurethanharnstoffs nicht verwendet.

Bevorzugt wird zur Herstellung des erfindungsgemäß verwendeten Polyurethanharnstoffs in einem Bereich von ≥ 0,5 und ≤ 20 Gew.-% der Summe der Komponenten C1. und gegebenenfalls E. oder bevorzugt in einem Bereich von ≥ 1 und ≤ 15 Gew.-% der Summe der Komponenten C1. und gegebenenfalls E., jeweils bezogen auf die Gesamtmasse des Polyurethanharnstoffs, eingesetzt.

Als Komponente F. können weitere polymere Polyole, welche unterschiedlich sind von B. eingesetzt werden.

Beispiele sind polymere Polyole, die nicht unter die Definition von B. fallen, weil sie keine Polyetherpolyole sind - beispielsweise die in der Polyurethanlacktechnologie an sich bekannten Polyesterpolyole, Polyacrylatpolyole, Polyurethanpolyole, Polycarbonatpolyole, , Polyesterpolyacrylatpolyole, Polyurethanpolyacrylatpolyole, Polyurethanpolyesterpolyole, , Polyurethanpolycarbonatpolyole und Polyesterpolycarbonatpolyole.

Bevorzugt handelt es sich bei der Komponente F. nicht um polymere Polyole die Estergruppen aufweisen, insbesondere nicht um Polyesterpolyole.

Die Komponenten B. und F. enthalten erfindungsgemäß zusammen ≤ 30 Gew.-%, bevorzugt ≤ 10 Gew.-%, und besonders bevorzugt ≤ 5 Gew.-%, an Komponente F., bezogen auf die Gesamtmasse der Komponenten B. und F.. Ganz besonders bevorzugt wird die Komponente F. zur Herstellung des Polyurethanharnstoffs nicht eingesetzt.

Bevorzugt wird zur Herstellung des erfindungsgemäß verwendeten Polyurethanharnstoffs in einem Bereich von ≥ 55 und ≤ 90 Gew.-% der Summe der Komponenten B. und gegebenenfalls F. und besonders bevorzugt in einem Bereich von ≥ 60 und ≤ 85 Gew.-% der Summe der Komponenten B. und gegebenenfalls F., jeweils bezogen auf die Gesamtmasse des Polyurethanharnstoffs, eingesetzt.

Bei der Komponente G. handelt es sich um Verbindungen, die genau eine isocyanatreaktive Gruppe aufweisen, oder um Verbindungen, die mehr als eine isocyanatreaktive Gruppe aufweisen, wobei nur eine der isocyanatreaktiven Gruppen unter den gewählten Umsetzungsbedingungen mit den in der Reaktionsmischung vorhandenen Isocyanatgruppen reagiert.

Die Komponente G. unterscheidet sich mindestens in einer Eigenschaft von den anderen isocyanatreaktiven Gruppen, insbesondere der Komponente B., die zur Herstellung der Polyurethan-Dispersion (B) eingesetzt werden. Bevorzugt unterscheidet sich die Komponente G. von den anderen isocyanatreaktiven Gruppen, insbesondere der Komponente B., aufgrund ihrer chemischen Zusammensetzung.

Bei den isocyanatreaktiven Gruppen der Komponente G. kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei insbesondere bevorzugt primäre oder sekundäre Aminogruppen, die mit Isocyanatgruppen unter Bildung von Harnstoffgruppen reagieren. Neben der Aminogruppe können die Verbindungen der Komponente G. auch andere prinzipiell isocyanatreaktive Gruppen, wie OH-Gruppen aufweisen, wobei nur eine der isocyanatreaktiven Gruppen unter den gewählten Umsetzungsbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente G. sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1- methylaminobutan, Ethanolamin, 3-Aminopropanol oder Neopentanolamin.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugt wird zur Herstellung des erfindungsgemäß verwendeten Polyurethanharnstoffs ≥ 0,1 und ≤ 20 Gew.-% der Komponente G. und besonders bevorzugt ≥ 0,3 und ≤ 10 Gew.-% der Komponente G., jeweils bezogen auf die Gesamtmasse des Polyurethanharnstoffs, eingesetzt.

Bevorzugt wird die Komponente H. eingesetzt und das molare Verhältnis von Komponente G. zu Komponente H. beträgt bevorzugt 5:1 bis 1:5, besonders bevorzugt 1,5:1 bis 1:4 und ganz besonders bevorzugt 1:1 bis 1:3.

Bevorzugt werden zur Herstellung der erfindungsgemäß verwendeten Polyurethanharnstoffe die Komponenten A. bis H. in den folgenden Mengen eingesetzt, wobei sich die Einzelmengen stets zu 100 Gew.-% addieren:
5 bis 40 Gew.-% Komponente A.,
55 bis 90 Gew.-% Summe der Komponenten B. und gegebenenfalls F.,
0,5 bis 20 Gew.-% Summe der Komponenten C1. und gegebenenfalls E.,
0,1 bis 10 Gew.-% Komponente C2.,
0 bis 20 Gew.-% Komponente D.,
0,1 bis 20 Gew.-% der Komponente G. und
0 bis 10 Gew.-% Komponente H..

Bevorzugt umfasst der geschäumte Artikel einen Polyurethanharnstoff, der erhältlich ist, durch Umsetzung von wenigstens
A. einer aliphatischen Polyisocyanat-Komponente mit einer mittleren Isocyanatfunktionalität von ≥ 1,8 und ≤ 2,6, welche ausgewählt ist aus HDI, IPDI und/oder H12-MDI oder deren Modifikationsprodukten, bevorzugt um ein Gemisch aus IPDI und HDI,
B. einer polymeren Polyether-Polyol-Komponente, welche aus Poly(tetramethylenglykol)polyetherpolyolen (wie (HO-(CHz -CH₂-CH₂-CH₂-O)ₓ-H) besteht, bevorzugt einer polymeren Polyether-Polyol-Komponente, welche ein Gemisch aus wenigstens zwei Poly(tetramethylenglykol)polyetherpolyolen ist und wobei sich die Poly(tetramethylenglykol)polyetherpolyole in ihren zahlenmittleren Molekulargewichten unterscheiden,
C. einer aminofunktionellen Kettenverlängerer-Komponente mit mindestens 2 isocyanatreaktiven primären und/oder sekundären Aminogruppen, enthaltend wenigstens eine aminofunktionelle Verbindung C1., die keine ionischen oder ionogenen Gruppen aufweist und/oder eine aminofunktionelle Verbindung C2., die ionische oder ionogene Gruppen aufweist,
D. gegebenenfalls weiteren hydrophilierenden Komponenten, die unterschiedlich sind von C2., wobei es sich bevorzugt um nichtionisch hydrophilierende Komponenten D 1. handelt
E. gegebenenfalls hydroxyfunktionellen Verbindungen mit einem Molekulargewicht von 62 bis 399 mol/g,
F. gegebenenfalls weiteren polymeren Polyolen, welche unterschiedlich sind von B.,
G. einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert, wobei es sich bei der isocyanatreaktiven Gruppe bevorzugt um eine primäre und/oder sekundäre Amino- und/oder Hydroxygruppe handelt und
H. gegebenenfalls einer aliphatischen Polyisocyanat-Komponente mit einer mittleren Isocyanatfunktionalität von > 2,6 und ≤ 4, wobei die Komponente H. aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur, bevorzugt basierend auf HDI, IPDI und/oder H12-MDI, besteht,
wobei die Komponenten B. und F. zusammen ≤ 30 Gew.-%, bevorzugt ≤ 20 Gew.-%, oder bevorzugt ≤ 10 Gew.-% an Komponente F., bezogen auf die Gesamtmasse der Komponenten B. und F. enthalten.

Das zahlenmittlere Molekulargewicht der erfindungsgemäß bevorzugt verwendeten Polyurethanharnstoffe beträgt bevorzugt von ≥ 2000 bis ≤ 300000 g/mol, bevorzugt von ≥ 5000 bis ≤ 150000 g/mol.

Der zur Herstellung des geschäumten Artikels eingesetzte Polyurethanharnstoff als Basis für die Polyurethanharnstoff-Dispersion (B) liegt bevorzugt in einem physiologisch akzeptablen Medium vor. Besonders bevorzugt handelt es sich bei dem Medium um Wasser und ganz besonders bevorzugt liegt der Polyurethanharnstoff als wässrige Dispersion (B) vor. Wasser bildet im Allgemeinen, neben wahlweise vorhandenen anderen flüssigen Medien, wie beispielsweise Lösungsmitteln, den Hauptbestandteil (> 50 Gew.-%) des Dispergiermediums, bezogen auf die Gesamtmenge des flüssigen Dispergiermediums, gegebenenfalls auch das alleinige flüssige Dispergiermedium.

Der geschäumte Artikel selbst enthält den Polyurethanharnstoff an sich, der keine oder nur noch Restmengen dieses Mediums enthält.

Bevorzugt ist der verwendete Polyurethanharnstoff daher in Wasser dispergierbar, was im Rahmen dieser Erfindung bedeutet, dass der Polyurethanharnstoff bei 23°C eine sedimentationsstabile Dispersion in Wasser, insbesondere entionisiertem Wasser, bilden kann.

Die verwendeten Polyurethanharnstoffe zur Bildung der Polyurethanharnstoff-Dispersion (B) sind bevorzugt erhältlich, indem isocyanatfunktionelle Polyurethanpräpolymere a) aus den Komponenten A., B. und gegebenenfalls D. und/oder C2., sowie gegebenenfalls den Verbindungen E. und/oder H. hergestellt werden (Schritt a) und deren freie NCO-Gruppen anschließend ganz oder teilweise mit der aminofunktionellen Kettenverlängerer-Komponente C., sowie der Komponente G. und gegebenenfalls der Komponente D. und H. umgesetzt werden (Schritt b).

Wobei wenn die Komponente H. erst in Schritt b) eingesetzt wird, diese bevorzugt vor der Zugabe der Komponente C. zugegeben und mit dem Prepolymer A. umgesetzt wird.

Bevorzugt erfolgt im Schritt b) die Umsetzung mit einem Diamin oder mehreren Diaminen (Komponente C. unter Kettenverlängerung wobei außerdem die monofunktionelle Komponente G. als Kettenabbrecher zur Molekulargewichtssteuerung zugesetzt wird.

Die Komponenten A. bis H. sind dabei wie oben angegeben definiert. Es gelten auch die oben genannten bevorzugten Ausführungsformen.

Bevorzugt wird im Schritt b) der Umsetzung des Präpolymers a) zur Herstellung des Polyurethanharnstoffs eine Mischung aus Komponenten C1., C2. und G. zur Umsetzung gebracht. Durch die Verwendung der Komponente C1. kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten C1., C2. und G. lässt sich eine optimale Balance zwischen Hydrophilie und Kettenlänge einstellen.

Bevorzugt weist das verwendete Polyurethanpräpolymere a) endständige Isocyanatgruppen auf, d.h. die Isocyanatgruppen liegen an den Kettenenden des Präpolymers. Besonders bevorzugt weisen sämtliche Kettenenden des Präpolymers Isocyanatgruppen auf.

Über die hydrophilierende Komponente C2. und/oder D. kann die Hydrophilie des Präpolymers gesteuert werden. Daneben spielen für die Hydrophilie des Präpolymers natürlich auch noch weitere Komponenten, speziell auch die Hydrophilie der Komponente B. eine Rolle.

Bevorzugt sind die isocyanatfunktionellen Polyurethanpräpolymere a) wasserunlöslich und nicht in Wasser dispergierbar.

Im Rahmen der Erfindung bedeutet der Begriff "wasserunlösliches, nicht wasserdispergierbares Polyurethanpräpolymer" insbesondere, dass die Wasserlöslichkeit des erfindungsgemäß verwendeten Präpolymers bei 23°C weniger als 10 g / Liter, bevorzugter weniger als 5 g / Liter beträgt und das Präpolymer bei 23° keine sedimentationsstabile Dispersion in Wasser, insbesondere entionisiertem Wasser, ergibt. Mit anderen Worten setzt sich das Präpolymer, beim Versuch es in Wasser zu dispergieren, ab. Die Wasserunlöslichkeit bzw. fehlende Dispergierbarkeit in Wasser bezieht sich auf entionisiertes Wasser ohne Zusatz von Tensiden.

Weiterhin weist das verwendete Polyurethanpräpolymere a) bevorzugt im Wesentlichen weder ionische noch ionogene (zur Bildung von ionischen Gruppen befähigte) Gruppen auf. Im Rahmen der vorliegenden Erfindung bedeutet dies, dass der Anteil der ionischen und/oder ionogenen Gruppen, wie insbesondere anionischer Gruppen, wie Carboxylat oder Sulfonat, oder kationischer Gruppen weniger beträgt als 15 Milliequivalente pro 100 g Polyurethanpräpolymer a), bevorzugt weniger als 5 Milliequivalente, besonders bevorzugt weniger als 1 Milliequivalent und ganz besonders bevorzugt weniger als 0,1 Milliequivalente pro 100 g Polyurethanpräpolymer a).

Im Falle saurer ionischer und/oder ionogener Gruppen beträgt die Säurezahl des Präpolymers zweckmäßig unter 30 mg KOH/g Präpolymer, bevorzugt unter 10 mg KOH/g Präpolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Dabei sind die wasserunlöslichen, nicht wasserdispergierbaren, isocyanatfunktionellen Polyurethanpräpolymeren A. bevorzugt ausschließlich erhältlich aus den Komponenten A., B. und gegebenenfalls D., E. und/oder H..

Die Komponenten sind dabei wie oben angegeben definiert. Es gelten auch die oben genannten bevorzugten Ausführungsformen.

In einer alternativen, weniger bevorzugten Ausführungsform der Erfindung sind die zur Herstellung des erfindungsgemäßen Polyurethanharnstoffs eingesetzten Präpolymere a) wasserlöslich oder wasserdispergierbar. In dieser Ausführungsform werden die hydrophilierende Komponente D. und/oder C2. bei der Herstellung des Präpolymers a) in einer Menge eingesetzt, die ausreichend ist, damit das Präpolymer wasserlöslich oder wasserdispergierbar ist. Das Präpolymer a) weist dabei bevorzugt ionische oder ionogene Gruppen auf.

Geeignete hydrophilierende Komponenten D. und C2. sind für diese Ausführungsform der Erfindung die oben für D. und C2. genannten Verbindungen. Bevorzugt werden als hydrophilierende Komponenten wenigstens die oben unter D1. und/oder C2. genannten Verbindungen eingesetzt.

Die zur Herstellung des geschäumten Artikels verwendeten Polyurethanharnstoffe werden bevorzugt vor, während oder nach Schritt b), besonders bevorzugt während oder nach Schritt b) in Wasser dispergiert. So wird eine Dispersion der Polyurethanharnstoffe, die Polyurethanharnstoff-Dispersion (B), erhalten.

Die Herstellung der Polyurethanharnstoff-Dispersionen (B) kann dabei in einer oder mehreren Stufe/-n in homogener oder bei mehrstufiger Umsetzung, teilweise in disperser Phase durchgeführt werden. Nach Herstellung des Präpolymers a) erfolgt bevorzugt ein Dispergier-, Emulgier- oder Lösungsschritt. Im Anschluss erfolgt gegebenenfalls eine weitere Polyaddition oder Modifikation in disperser Phase. Dabei wird jeweils das für das entsprechende Präpolymer geeignete Löse- oder Dispergiermittel wie beispielsweise Wasser oder Aceton oder Mischungen daraus gewählt.

Dabei können alle aus dem Stand der Technik bekannten Verfahren wie z. B. Präpolymer-Mischverfahren, Acetonverfahren oder Schmelzdispergierverfahren verwendet werden. Bevorzugt wird das Aceton-Verfahren angewandt.

Für die Herstellung nach dem Aceton-Verfahren werden üblicherweise die Bestandteile B., gegebenenfalls D. und E. und die Polyisocyanatkomponente A. gegebenfalls in Kombination mit der Komponente H. zur Herstellung eines isocyanatfunktionellen Polyurethanpräpolymers ganz oder teilweise vorgelegt und gegebenenfalls mit einem mit Wasser mischbaren aber gegenüber Isocyanat-gruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend können die gegebenenfalls zu Beginn der Reaktion noch nicht zugegebenen Bestandteile von A., B. und gegebenenfalls H., D. und E. zu dosiert werden.

Bei der Herstellung des Polyurethan-Präpolymeren aus A., B. und gegebenenfalls H., D. und E. beträgt das Stoffmengenverhältnis von Isocyanat-Gruppen zu isocyanatreaktiven Gruppen bevorzugt 1,05 bis 3,5, besonders bevorzugt 1,1 bis 3,0 und ganz besonders bevorzugt 1,1 bis 2,5.

Die Umsetzung der Komponenten A., B. und gegebenenfalls H., D. und E. zum Präpolymer kann teilweise oder vollständig, bevorzugt aber vollständig erfolgen. Es können so Polyurethanpräpolymere, die freie Isocyanatgruppen enthalten, in Substanz oder in Lösung erhalten werden.

Sollten ionogene Gruppen, wie beispielsweise Carboxylgruppen, im Präpolymer vorliegen können diese in einem weiteren Schritt durch Neutralisation in ionische Gruppen überführt werden.

Im Neutralisationsschritt zur teilweisen oder vollständigen Überführung potentiell anionischer Gruppen in anionische Gruppen können Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt werden.

Als Neutralisationsmittel sind bevorzugt anorganische Basen, wie wässrige Ammoniaklösung oder Natrium- bzw. Kaliumhydroxid einsetzbar, besonders bevorzugt sind Natriumhydroxid und Kaliumhydroxid.

Die Stoffmenge der Basen beträgt bevorzugt 50 und 125 mol%, besonders bevorzugt zwischen 70 und 100 mol% der Stoffmenge der zu neutralisierenden Säuregruppen. Die Neutralisation kann, auch gleichzeitig mit der Dispergierung erfolgen, in dem das Dispergierwasser bereits das Neutralisationsmittel enthält.

Im Anschluss an die Neutralisation wird in einem weiteren Verfahrensschritt, falls noch nicht oder nur teilweise geschehen, das erhaltene Präpolymer mit Hilfe von aliphatischen Ketonen wie Aceton oder 2-Butanon gelöst.

Bei der Kettenverlängerung/-terminierung in Stufe b) werden die Komponenten C., G. und gegebenenfalls D. mit den noch verbliebenen Isocyanatgruppen des Präpolymers umgesetzt. Bevorzugt wird die Kettenverlängerung/-terminierung vor der Dispergierung in Wasser durchgeführt.

Geeignete Komponenten C. zur Kettenverlängerung sowie G. zum Kettenabbruch sind bereits oben aufgeführt. Es gelten analog auch die oben genannten bevorzugten Ausführungsformen.

Werden zur Kettenverlängerung anionische Hydrophilierungsmittel entsprechend der Definition C2. mit NH₂- oder NH-Gruppen eingesetzt, erfolgt die Kettenverlängerung der Präpolymere in Schritt b) bevorzugt vor der Dispergierung in Wasser.

Das Äquivalentverhältnis von NCO-reaktiven Gruppen der zur Kettenverlängerung und Kettenterminierung eingesetzten Verbindungen zu freien NCO-Gruppen des Präpolymers, liegt im allgemeinen zwischen 40 und 150 %, bevorzugt zwischen 50 und 110 %, besonders bevorzugt zwischen 60 und 100 %.

Die Komponenten C1., C2. und G., können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel in Schritt b) mit verwendet werden, so beträgt der jeweilige Verdünnungsmittelgehalt in den eingesetzten Komponenten C1., C2. und G. bevorzugt 40 bis 95 Gew.-%.

Die Dispergierung erfolgt bevorzugt im Anschluss an die Kettenverlängerung und Kettenterminierung. Dazu wird das gelöste (beispielsweise in Aceton) und mit dem Aminen umgesetzte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerten Polyurethanpolymerlösungen gerührt. Bevorzugt wird das Wasser in das gelöste Polyurethanpolymer gegeben. Auf diese Weise wird die Polyurethanharnstoff-Dispersion (B) erhalten.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Die erhaltenen wässrigen Polyurethanharnstoff-Dispersionen (B) weisen bevorzugt einen Gehalt an flüchtigen organischen Verbindungen (VOC), wie beispielsweise flüchtige organische Lösemitteln, von weniger als 10 Gew.-%, bevorzugter von weniger als 3 Gew.-%, noch bevorzugter von weniger als 1 Gew.-% bezogen auf die auf wässrige Polyurethanharnstoffdispersion auf. VOCs im Sinne dieser Erfindung sind insbesondere organische Verbindung mit einem Anfangssiedepunkt von höchstens 250 °C bei einem Standarddruck von 101,3 kPa.

Die Bestimmung des Gehalts an flüchtigen organischen Verbindungen (VOC) erfolgt im Rahmen der vorliegenden Erfindung insbesondere durch gaschromatographische Analyse.

Der pH-Wert der erfindungsgemäß verwendeten wässrigen Polyurethanharnstoff-Dispersion (B) beträgt typischerweise weniger als 9,0, bevorzugt weniger als 8,5 und liegt besonders bevorzugt zwischen 5,5 und 8,0.

Um eine gute Sedimentationsstabilität zu erreichen, liegt die zahlenmittlere Teilchengröße der speziellen Polyurethanharnstoff-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malvern Inst. Limited).

Der Feststoffgehalt der Polyurethanharnstoff-Dispersionen (B) beträgt bevorzugt 10 bis 70 Gew.-%, besonders bevorzugt 20 bis 60 Gew.-%und ganz besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethanharnstoff-Dispersionen (B) weniger als 5 Gew.-%, besonders bevorzugt weniger als 0,2 Gew.-%, bezogen auf die Masse der Dispersionen (B), an ungebundenen organischen Aminen auf.

Die zur Herstellung der geschäumten Artikel eingesetzte Polyurethanharnstoff-Dispersion (B) weist bei 23°C bei einer konstanten Scherrate von 10 s⁻¹ bevorzugt eine Viskosität von ≥ 1 und ≤ 10000 mPa s, oder bevorzugt von ≥ 10 und ≤ 5000 mPa s und besonders bevorzugt von ≥ 100 und ≤ 4000 mPa s auf. Die Viskosität wird dabei wie im Methodenteil beschrieben bestimmt.

Bevorzugt sind die beiden Polyurethandispersionen (A) und (B) chemisch oder physikalisch nicht identisch. Die beiden Polyurethandispersionen (A) und (B) unterscheiden sich bevorzugt in der Höhe der Molekularmassen der enthaltenen Polyurethane enthalten in (A) und (B) voneinander. Der Unterschied des mittleren Molekulargewichtes Mw (ermittelt per GPC mit DMAc als Laufmittel) der Polyurethane von (A) zu (B) liegt bevorzugt in einem Bereich von 20:1 bis 1,2:1, besonders bevorzugt von 10:1 bis 1,3:1 und ganz besonders bevorzugt von 5:1 bis 1,5:1. Das Polyurethanpolymer der Polyurethandispersion (A) hat bevorzugt ein mittleres Molekulargewicht Mw in einem Bereich von 150.000 bis 1.000.000 g/mol, oder bevorzugt von 170 000 bis 700 000 g/mol, besonders bevorzugt von 180 000 bis 500 000 g/mol. Das Polyurethanpolymer der Polyurethandispersion (B) hat bevorzugt ein mittleres Molekulargewicht Mw in einem Bereich von 10 000 bis 170 000 g/mol, oder bevorzugt von 20 000 bis 150 000 g/mol.

Neben den Polyurethan-Dispersionen (A) und (B) können auch Additive in Form von Hilfs- und Zusatzstoffen in dem geschäumten Artikel mitverwendet werden.

Beispiele für solche Hilfs- und Zusatzstoffe sind Schaumhilfsmittel wie Schaumbildner und -stabilisatoren, Verdicker bzw. Thixotropiermittel, Antioxidantien, Lichtschutzmittel, Emulgatoren, Weichmacher, Pigmente, Füllstoffe, Farbstoffe, Additive zur Gebindestabilisierung, Biozide, pH-Regulatoren, Dispersionen und/oder Verlaufshilfsmittel. Je nach gewünschtem Eigenschaftsbild und Verwendungszweck der geschäumten Artikel können ≤ 30 Gew.-%, bevorzugt ≤ 20 Gew.-%, oder bevorzugt ≤ 10 Gew.-%, bezogen auf Gesamttrockensubstanz des geschäumten Artikels, solcher Füllstoffe im Endprodukt enthalten sein. Bevorzugt werden keine Farbstoffe oder Pigmente eingesetzt, sodass bevorzugt weiße Schäume und damit weiße geschäumte Artikel entstehen.

Bevorzugt sind als Hilfs- und Zusatzstoffe Schaumhilfsmittel wie Schaumbildner und -stabilisatoren enthalten. Als Schaumstabilisatoren bezeichnet man Zusatzstoffe, die die Lebensdauer von Schäumen durch Erhöhen der Grenzflächenviskosität und Grenzflächenelastizität der Schaumlamellen verlängern, wie in "spektrum.de", Lexikon der Chemie beschrieben. Generell sind als Schaumstabilisatoren alle grenzflächenaktiven amphiphilen Substanzen einsetzbar. Besonders geeignet sind beispielsweise handelsübliche Verbindungen wie Fettsäureamide, Sulfosuccinamide, Kohlenwasserstoffsulfonate, -sulfate oder Fettsäuresalze, wobei der lipophile Rest bevorzugt 12 bis 24 Kohlenstoffatome enthält, amphiphile Organosilicon-Copolymere sowie Alkylpolyglycoside, die nach dem Fachmann an sich bekannten Methoden durch Umsetzung von längerkettigen Monoalkoholen (4 bis 22 C-Atome im Alkylrest) mit Mono-, Di- oder Polysacchariden erhältlich sind (siehe z.B. Kirk-Othmer, Encyclopedia of Chemical Technology, John Wiley & Sons, Vol. 24, S. 29). Besonders geeignete Schaumhilfsmittel sind EO/PO-Blockcopolymere, die nach dem Fachmann an sich bekannten Methoden durch Addition von Ethylenoxid und Propylenoxid an OH- oder NH-funktionelle Startermoleküle erhältlich sind (siehe z.B. Kirk-Othmer, Encyclopedia of Chemical Technology, John Wiley & Sons, Vol. 24, S. 28). Zur Verbesserung der Schaumbildung, Schaumstabilität oder der Eigenschaften des resultierenden Polyurethan-Schaums, können neben oder alternativ zu den EO/PO-Blockcopolymeren weiteren Additive in der Mischung enthalten sein. Solche weiteren Additive können grundsätzlich alle an sich bekannten anionischen, nichtionischen und kationischen Tenside sein, bevorzugt sind anionische und nicht-ionische Tenside, besonders bevorzugt sind nichtionische Tenside. Tenside sind Substanzen, die die Oberflächenspannung einer Flüssigkeit oder die Grenzflächenspannung zwischen zwei Phasen herabsetzen. Bestimmte Verbindungen können folglich sowohl als Tensid als auch als Schaumstabilisator wirken. Insbesondere bevorzugt werden als schaumbildende Hilfsmittel jedoch allein amphiphile, nichtionische EO/PO-Blockcopolymere als Additive eingesetzt. Bevorzugt werden 0,5 bis 5 Gew.-%, oder bevorzugt 1 bis 4 Gew.-%, besonders bevorzugt 1,5 bis 3,4 Gew.-% an Schaumstabilisator, bezogen auf die Gesamtmasse der Polyurethandispersionen (A) und (B) eingesetzt. Wie später noch im Detail erläutert sind Schaumstabilisatoren bevorzugt, die eine möglichst geringe bis keine zytotoxische Aktivität aufweisen, was in Form der Viabilität von Zellen in einem Zytotoxtest ermittelt werden kann. Bevorzugt weisen die Schaumstabilisatoren eine Viabilität der Zellen von ≧70% bzw. eine Klassifizierung als 0-2 (no to mild cytotoxicity) in einem Zytotoxtest nach DIN ISO 10993-5 auf.

Als Verdicker können handelsübliche Verdicker eingesetzt werden, wie Dextrin-, Stärke-, Polysaccharidderivate wie Guargummi oder Cellulosederivate wie Celluloseether oder Hydroxyethylcellulose, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren. Besonders bevorzugt werden assoziative Polyurethanverdicker eingesetzt. Die Mengen an Verdicker liegt bevorzugt bei ≤ 5 Gew.-%, oder bevorzugt in einem Bereich von 0,1 bis 5 Gew.-%, weiter bevorzugt von 0,2 bis 2 Gew.-%, bezogen auf die Gesamtmasse aus der Summe der Dispersionen (A) und (B). Bevorzugt werden Verdicker eingesetzt, die eine möglichst geringe bis keine zytotoxische Aktivität aufweisen, was in Form der Viabilität von Zellen in einem Zytotoxtest ermittelt werden kann.

Weiterhin ist es bevorzugt möglich bestimmte Vernetzer zur Herstellung des geschäumten Artikels zu verwenden, um beispielsweise die chemische Resistenz des geschäumten Artikels gegenüber Basen und Säuren oder Lösungsmitteln zu verbessern. Beispiele hierfür sind polyisocyanat-basierte Vernetzer wie Bayhydur^{®} 3100, Baymedix^{®} FP520. Der geschäumte Artikel beinhaltet Vernetzer bevorzugt in einer Mengen in einem Bereich von 0 bis 5 Gew.-%, oder bevorzugt von 0,1 bis 4,5 Gew.-%, oder bevorzugt in einem Bereich von 0,5 bis 3 Gew.-%, bezogen auf die Gesamtmasse des geschäumten Artikels. Ebenfalls möglich ist die Zugabe, Einarbeitung oder Beschichtung des geschäumten Artikels von bzw. mit antimikrobiellen oder biologischen Wirkstoffen, welche sich beispielsweise in Bezug auf die Wundheilung und die Vermeidung von Keimbelastungen positiv auswirken. Die Zugabe oder Einarbeitung mit diesen Wirkstoffen kann sowohl durch Zugabe in die Polyurathan-Dispersion (A) und/oder in die Polyurethanharnstoff-Dispersion (B) erfolgen.

Bevorzugte Wirkstoffe der vorgenannten Art sind solche aus der Gruppe der Antiseptika, Wachstumsfaktoren, Proteaseinhibitoren und nichtsteroidalen Antiphlogistika/Opiate oder auch Wirkstoffe wie beispielsweise Thrombin alfa zur lokalen Blutstillung.

Bevorzug umfasst der Wirkstoff zumindest ein Bakteriostatikum oder ein Bakterizid, ganz besonders bevorzugt ein antiseptisches Biguanid und/oder dessen Salz, vorzugsweise das Hydrochlorid.

Beispiele für Mischungen zur Herstellung des erfindungsgemäßen Artikels werden nachfolgend aufgeführt, wobei die Summe der Angaben in Gew.-% einen Wert von ≤ 100 Gew.-% einnimmt. Diese Mischungen umfassen, bezogen auf die Gesamtmasse der Mischung, typischerweise ≥ 80 bis ≤ 100 Gew.-% der Dispersionen (A) und (B) in Summe, ≥ 0 bis ≤ 10 Gew.-% Schaumhilfsmittel, ≥ 0 bis ≤ 10 Gew.-% Vernetzer und ≥ 0 bis ≤ 10 Gew.-% Verdicker.

Bevorzugt umfasst die Mischung, bezogen auf die Gesamtmasse der Mischung, ≥ 85 bis ≤ 100 Gew.-% der Dispersionen (A) und (B) in Summe, ≥ 0 bis ≤ 7 Gew.-% Schaumhilfsmittel, ≥ 0 bis ≤ 5 Gew.-% Vernetzer, ≥ 0 bis ≤ 10 Gew.-% Antiseptika bzw. Biozide und ≥ 0 bis ≤ 5 Gew.-% Verdicker.

Besonders bevorzugt umfasst die Mischung, bezogen auf die Gesamtmasse der Mischung, ≥ 89 bis ≤ 100 Gew.-% der Dispersionen (A) und (B) in Summe, ≥ 0 bis ≤ 6 Gew.-% Schaumhilfsmittel, ≥ 0 bis ≤ 4 Gew.-% Vernetzer und ≥ 0 bis ≤ 4 Gew.-% Verdicker.

Bevorzugt ist der geschäumte Artikel rissfrei. Unter rissfrei wird gemäß der Erfindung verstanden, dass mindestens eines, bevorzugt mindestens zwei, besonders bevorzugt alle der folgenden Kriterien für den geschäumten Artikel erfüllt sind:
i. der geschäumte Artikel keine Risse aufweist, die eine Breite von ≥ 10 mm, bevorzugt von ≥ 5 mm, mehr bevorzugt von ≥ 2 mm, besonders bevorzugt von ≥ 0,5 mm aufweisen;
ii. der geschäumte Artikel auf seiner Oberfläche keine Risse hat, die tiefer als 0,4 mm sind;
iii. der geschäumte Artikel eine Rissfläche von ≤ 6%, bevorzugt ≤ 3 % oder bevorzugt ≤ 0,5 % aufweist, bezogen auf die Gesamtoberfläche des geschäumten Artikels;

Die Rissbreite unter i., die Risstiefe unter ii. und die Rissfläche unter iii. wird jeweils gemäß der Methode, wie weiter unten in den Messmethoden beschrieben, bestimmt.

In einer bevorzugten Ausgestaltung des geschäumten Artikels weist der geschäumte Artikel mindestens eine, bevorzugt mindestens zwei, oder bevorzugt alle der folgenden Eigenschaften i. bis xiii. auf:
i. der geschäumte Artikel keine Risse aufweist, die eine Breite von ≥ 2 mm, bevorzugt von ≥ 1 mm, mehr bevorzugt von ≥ 0,5 mm, besonders bevorzugt von ≥ 0,1 mm aufweisen, wobei die Rissbreite gemäß der Methode unter Messmethoden bestimmt wurde;
ii. der geschäumte Artikel auf seiner Oberfläche keine Risse hat, die tiefer als 0,4 mm, bevorzugt tiefer als 0,2 mm, oder bevorzugt tiefer als 0,1 mm sind, wobei die Risstiefe gemäß der Methode unter Messmethoden bestimmt wurde;
iii. der geschäumte Artikel eine Rissfläche von ≤ 6%, bevorzugt ≤ 3 % oder bevorzugt ≤ 0,5 % aufweist, bezogen auf die Gesamtoberfläche des geschäumten Artikels;
iv. der geschäumte Artikel eine Viabilität der Zellen von ≥ 70% bzw. Klassifizierung als 0-2 (no to mild cytotoxicity) in einem Zytotoxtest nach DIN ISO 10993-5 aufweist;
v. der geschäumte Artikel eine Dichte in einem Bereich von 80 bis 500 g/L, bevorzugt 100 bis 400 g/L, oder bevorzugt 120 bis 300 g/L aufweist;
vi. der geschäumte Artikel eine Dicke (D) in einem Bereich von 0,1 bis 100 mm, bevorzugt in einem Bereich von 0,5 bis 50 mm, bevorzugt in einem Bereich von 1 bis 10 mm aufweist;
vii. der geschäumte Artikel eine Bruchspannung in einem Bereich von 100 bis 1100 kPa, bevorzugt in einem Bereich von 200 bis 500 kPa, nach DIN EN ISO 527-2:2012-06 gemessen, aufweist;
viii. der geschäumte Artikel eine Bruchdehnung in einem Bereich von 100 bis 500 %, bevorzugt in einem Bereich von 150 bis 400 %, nach DIN EN ISO 527-2:2012-06 gemessen, aufweist;
ix. dass der Polyurethanharnstoff gebildet aus der Polyurethandispersion (B) amorph ist und einen Tg ≤ - 25 °C, bevorzugt ≤ - 40 °C, oder bevorzugt ≤ - 50 °C aufweist, bestimmt mittels dynamischer Differenzkalorimetrie in Anlehnung an die DIN EN 61006, Verfahren A;
x. der geschäumte Artikel einen Schmelz- oder Erweichungsbereich von ≤ 180°C, bevorzugt ≤ 150°C bei einem maximalen Druck von 4 bar hat und in diesem Bereich vollständig thermoplastisch verarbeitbar ist;
xi. der geschäumte Artikel eine mittlere Porengröße in einem Bereich von 200 bis 750 µm, bevorzugt in einem Bereich von 300 bis 600 µm hat;
xii. der geschäumte Artikel eine Deckschicht, bevorzugt ein thermoplastisches Polyurethan oder ein thermoplastisches Polyethylen enthaltend, mindestens auf einer Oberfläche des geschäumten Artikels aufweist;
xiii. dass der geschäumte Artikel Bestandteil eines Verbundmaterials ist.

Bevorzugt weist der geschäumte Artikel mindestens die Eigenschaften i. und ii., besonders bevorzugt die Eigenschaften i. ii. und iii. auf. Weiterhin bevorzugt ist es, wenn der geschäumte Artikel mindestens die Eigenschaften i. und iv., oder ii. und iv., oder iii. und iv., oder ganz besonders bevorzugt die Eigenschaften i., ii., iii. und iv. aufweist.

Unter "thermoplastisch verarbeitbar" wird verstanden, dass eine Verformung des Materials bei Erhitzen stattfinden kann bzw. eine Verarbeitung wie Laminieren ohne dabei die Grundstruktur des zugrundeliegenden Polymers dabei zu zerstören, da bei thermoplastischen Materialien beim moderaten Erhitzen lediglich ein Ausschmelzen der Struktur mit anschließendem Kristallisieren beim Abkühlen stattfindet.

Amorph bedeutet im Sinne dieser Erfindung, dass der Polyurethanharnstoff im, in der im folgenden ausgeführten Messmethode genannten, Temperaturbereich keine oder nur so geringe kristalline Anteile ausbildet, dass mittels der beschriebenen DSC Messungen nur ein oder mehrere Glasübergangspunkte T_{g}, aber keine Schmelzbereiche mit einer Schmelzenthalpie ≥ 20J/g in dem genannten Temperaturbereich gefunden werden können.

Die Glasübergangstemperatur T_{g} wird im Rahmen dieser Erfindung mittels dynamischer Differenzkalorimetrie in Anlehnung an die DIN EN 61006, Verfahren A, bestimmt, wobei ein DSC Gerät verwendet wird, das zur Bestimmung von T_{g} mit Indium und Blei kalibriert ist und wobei drei unmittelbar aufeinander folgende Durchläufe einer Aufheizungen von -100°C bis +150°C, bei einer Heizrate von 20 K/min, mit anschließender Abkühlung mit einer Kühlrate von 320 K/min vorgenommen und die dritte Aufheizkurve zur Bestimmung der Werte verwendet wird und wobei als T_{g} die Temperatur bei der halben Höhe einer Glasübergangsstufe bestimmt wird.

Sollte der Polyurethanharnstoff in Form einer Dispersion vorliegen, wird bei der Probenpräparation für die DSC Messungen besonders vorgegangen. Bei der Bestimmung der Glasübergangstemperatur T_{g} von Dispersionen mittels DSC kann die T_{g} des Polymers durch die kalorischen Effekte des Dispergens (Wasser, Neutralisationsmittel, Emulgatoren, Colöser, etc.) maskiert bzw. wegen der Mischbarkeit mit dem Polymer deutlich abgesenkt werden. Daher wird das Dispergens vor der DSC-Messung bevorzugt durch geeignete Trocknung zunächst vollständig entfernt, denn auch geringe Restmengen Dispergens wirken als Weichmacher und können die Glastemperatur dadurch absenken. Die Dispersion wird daher bevorzugt mit 100 µm Nassschichtdicke (NSD) auf eine Glasplatte gerakelt, abgelüftet und dann für zwei Tage in einer Trockenbox bei RT und 0% relativer Luftfeuchtigkeit (rF) getrocknet. Nach dieser Probenpräparation kann in der ersten Aufheizung der DSC Messung noch Restfeuchte im Film einen breiten endothermen Verdampfungsbereich erzeugen. Um die bestimmten Werte möglichst von solchen Einflüssen frei zu erhalten, wird daher die dritte Aufheizkurve ausgewertet.

In einer bevorzugten Ausgestaltung des geschäumten Artikels liegt das Gewichts-Verhältnis der Polyurethan-Dispersion (A) zu der Polyurethan-Dispersion (B) in einem Bereich von 1:1 bis 5:1, bevorzugt in einem Bereich von 1:1 bis 4:1, oder bevorzugt in einem Bereich von 1:1 bis 3:1, bezogen auf die Gesamtmasse aus den Massen der Dispersionen (A) und (B).

In einer bevorzugten Ausgestaltung des geschäumten Artikels weist ein durch Trocknung aus der Dispersion (B) gebildeter Film eine Bruchspannung von ≤ 5 MPa, bevorzugt ≤ 3,5 MPa und besonders bevorzugt ≤ 2,5 MPa. Der aus der Dispersion (B) gebildete Film weist bevorzugt eine Bruchdehnung von ≥ 1750 %, bevorzugt ≥ 2000 %, bevorzugt in einem Bereich von≥ 1750 bis 3000 %, besonders bevorzugt von 2000 bis 4000 % auf. Bevorzugt ist der 500 % Modul des aus Dispersion (B) gebildeten Films bei bevorzugt ≤ 2 MPa, oder bevorzugt ≤ 1,5 MPa oder bevorzugt ≤ 1,0 MPa, besonders bevorzugt in einem Bereich von 0,1 bis 2 MPa.

Für die Herstellung der benötigten Filme aus der Dispersion (A), der Dispersion (B) oder einem Gemisch aus den Dispersionen (A) und (B) werden mit einem Handrakel mit einer Spaltbreite von 200 µm die jeweilige Dispersion auf einem Polyolefin-beschichteten Trennpapier aufgetragen und 20min bei 40°C und anschließend 10min bei 130°C im Umlufttrockenschrank getrocknet.

Bevorzugt ist der Polyurethanharnstoff, gebildet aus der Polyurethandispersion (B), amorph und weist einen Tg ≤ - 25 °C, bevorzugt ≤ - 40 °C, oder bevorzugt ≤ - 50 °C auf, bestimmt mittels dynamischer Differenzkalorimetrie gemäß DIN EN 61006, Verfahren A.

Es hat sich überraschenderweise herausgestellt, dass gerade durch die geeignete Mischung einer schaumbildenden Dispersion (A) mit einer Dispersion (B), aus der Filme gebildet werden können, die als sehr weich bezeichnet werden können, ein geschäumter Artikel hergestellt werden kann, der rissfrei ist.

Ein Film gebildet aus Dispersion (A) weist bevorzugt eine Bruchdehnung im Bereich von 400 % bis 700 %, eine Bruchspannung von 20 bis 50 MPa und einen 100% Modul von bevorzugt ≤ 7 MPa, bevorzugt in einem Bereich von 2 bis 7 MPa auf. Das Verfahren zur Herstellung des Films aus Dispersion (A) wird später im Detail beschrieben.

Bevorzugt weist ein aus der Dispersion (A) gebildeter Film eine Bruchdehnung auf, die in einem Bereich von 1 bis 50 % der Bruchdehnung eines aus der Dispersion (B) gebildeten Films liegt. Bevorzugt weist ein aus der Dispersion (A) gebildeter Film eine Bruchspannung auf, die in einem Bereich von 5 bis 40 % der Bruchspannung eines aus der Dispersion (B) gebildeten Films liegt. Bevorzugt weist ein aus der Dispersion (A) gebildeter Film einen 100% Modul auf, das in einem Bereich von 10 bis 50 % des 100% Moduls eines aus der Dispersion (B) gebildeten Films liegt.

Die Modulwerte und Elongationen zur Bestimmung der Bruchspannung und der Bruchdehnung wurden in einem Zugprüfungsverfahren in Anlehnung an DIN 53504:2009-10 mit einer Prüfgeschwindigkeit von 200 mm/min und einer Vorkraft von 0,05 N/mm² bestimmt.

In einer bevorzugten Ausgestaltung des geschäumten Artikels werden als Hydrophilierungsmittel B2) Sulfongruppen-haltige Hydrophilierungsmitteln verwendet. Bevorzugt wird als Sulfongruppenhaltiges Hydrophilierungsmittel das Natrium- oder Kaliumsalz der Hydroxyethansulfonsäure verwendet.

In einer bevorzugten Ausgestaltung des geschäumten Artikels weist die Dispersion (A) einen Feststoffgehalt an Polyurethan von 52 bis 65 Gew.-%, bevorzugt von 55 bis 63 Gew.-%, oder bevorzugt von 57 bis 62 Gew.-%, bezogen auf die Gesamtmasse der Dispersion (A) auf.

In einer bevorzugten Ausgestaltung des geschäumten Artikels ist die Komponente A. oder A1) Isophorondiisocyanat (IPDI) und/oder Hexamethylendiisocyanat (HDI). Weiterhin bevorzugt ist die Komponente A. oder A1) ein Gemisch aus IPDI und HDI, bevorzugt in einem Mengenverhältnis in einem Bereich von 2:1 bis 1:2, oder bevorzugt in einem Bereich von 1,5:1 bis 1:1,5.

In einer bevorzugten Ausgestaltung des geschäumten Artikels enthält die Komponente B. Poly(tetramethylenglykol)polyetherpolyole oder besteht daraus.

In einer bevorzugten Ausgestaltung des geschäumten Artikels enthält die Komponente B. ein Gemisch aus mindestens zwei Poly(tetramethylenglykol)polyetherpolyolen oder besteht daraus, wobei sich die mindestens zwei Poly(tetramethylenglykol)polyetherpolyole in ihren zahlenmittleren Molekulargewichten unterscheiden. Bevorzugt enthält die Komponente B. ein Gemisch aus Poly(tetramethylenglykol)polyetherpolyolen I mit einem zahlenmittleren Molekulargewichte Mₙ in einem Bereich von ≥ 400 und ≤ 1500 g/mol, besonders bevorzugt in einem Bereich von ≥ 600 und ≤ 1200 g/mol, ganz besonders bevorzugt in einem Bereich von 1000 g/mol und Poly(tetramethylenglykol)polyetherpolyole II mit einem zahlenmittleren Molekulargewichte Mₙ in einem Bereich von ≥ 1500 und ≤ 8000 g/mol, besonders bevorzugt in einem Bereich von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylenglykol)polyetherpolyolen II liegt bevorzugt im Bereich von 0,1 bis 10, besonders bevorzugt im Bereich von 0,2 bis 10, ganz besonders bevorzugt im Bereich von 1 bis 6.

In einer bevorzugten Ausgestaltung des geschäumten Artikels handelt es sich bei der Komponente D. um nichtionisch hydrophilierende Komponenten D1. Bevorzugt werden als nichtionisch hydrophilierende Komponente D1. nichtionische monofunktionelle gemischte Polyalkylenoxidpolyether, die 40 bis 100 mol-% Ethylenoxid- und 0 bis 60 mol-% Propylenoxideinheiten, verwendet, wie sie bereits zuvor beschrieben wurde.

In einer bevorzugten Ausgestaltung des geschäumten Artikels wird die Komponente H. eingesetzt und das molare Verhältnis von Komponente G. zu Komponente H. beträgt 5:1 bis 1:5, oder bevorzugt 4,5:1 bis 1:4,5, oder bevorzugt 4:1 bis 1:4.

In einer bevorzugten Ausgestaltung des geschäumten Artikels ist die Polyurethandispersion (B) erhältlich indem isocyanatfunktionelle Polyurethanpräpolymere a) aus den Komponenten A., B. und gegebenenfalls D. und/oder C2., sowie gegebenenfalls den Verbindungen E. und/oder H. hergestellt werden und deren freie NCO-Gruppen anschließend ganz oder teilweise mit der aminofunktionellen Kettenverlängerer-Komponente C., sowie der Komponente G. und gegebenenfalls der Komponente D. und H. umgesetzt werden.

Ein weiterer Gegenstand der Erfindung ist ein Verfahren zur Herstellung eines erfindungsgemäßen, geschäumten Artikels, wobei das Verfahren mindestens die folgenden Schritte enthält:
(V1) Mischen der Polyurethandispersion (A) und der Polyurethandispersion (B) unter Erhalt einer Mischung (M1),
(V2) ggf. Zugabe zu (M1) mindestens eines Grenzflächen-aktiven Stoffes, wie ein Schaumstabilisator, der bevorzugt nicht-zytotoxisch ist,
(V3) ggf. Zugabe zu (M1) mindestens eines Tensid (flüssig/flüssig oder flüssig/Luft), das bevorzugt nicht-zytotoxisch ist,
(V4) ggf. Zugabe zu (M1) eines Verdicker, bevorzugt eines assoziativen PU-Verdicker, der bevorzugt nicht-zytotoxisch ist,
(V5) Aufschäumen der Mischung (M1) zu einem Schaum, bevorzugt durch Einbringen von Luft in die Mischung (M1)
(V6) ggf. Reifen des Schaums aus Schritt (V2),
(V7) ggf. Aufbringen des in Schritt (V5) oder (V6) gebildeten Schaums auf ein Substrat,
(V8) Trocknen des Schaums unter Bildung des geschäumten Artikels.

Das Mischen der Dispersionen (A) und (B) in Schritt (V1) kann mit jedem Gerät und auf jede erdenkliche Art und Weise erfolgen, die der Fachmann zur homogenen Vermischung von Polyurethandisperionen unter Erhalt einer Mischung (M1) verwenden würde. Bevorzugt wird das Mischen in Schritt (V1) in einem Behältnis aus Edelstahl durchgeführt. Das Mischen in Schritt (V 1) findet bevorzugt über einen Zeitraum in einem Bereich von 30 Sekunden bis 60 Minute, oder bevorzugt in einem Bereich von 1 bis 30 Minuten statt.

Die optionale Zugabe mindestens eines Grenzflächen-aktiven Stoffes, wie ein Schaumstabilisator in Schritt (V2) sowie optional eines Tensids in Schritt (V3) und optional eines Verdickers in Schritt (V4) kann durch jedes Hilfsmittel und auf jede Art erfolgen, die der Fachmann für eine Zugabe von solchen Additiven, wie sie bereits zuvor für den erfindungsgemäßen geschäumten Artikel beschrieben wurden, zu Polyurethandispersionen und deren Mischungen auswählen würde.

Bevorzugt erfolgt die Zugabe des mindestens einen Grenzflächen-aktiven Stoffes in Schritt (V2) durch Zugabe dieses Stoffes in eine oder beider der Dispersionen (A) oder (B) vor Schritt (V1). Genauso kann die optionale Zugabe des Tensids (V3) sowie die optionale Zugabe des Verdickers in Schritt (V4) in eine oder beider der Dispersionen (A) oder (B) vor Schritt (V1) erfolgen.

Alternativ oder zusätzlich kann die Zugabe des mindestens einen Grenzflächen-aktiven Stoffes oder des Tensids (V3) sowie die optionale Zugabe des Verdickers (V4) während des Mischens in Schritt (V1) oder im Anschluss an das Mischen in Schritt (V1) erfolgen. Es können auch mehrere Grenzflächen-aktive Stoffe und/oder mehrere Tenside und/oder mehrere Verdicker in Schritt (V2), (V3) und/oder (V4) in die Mischung aus (V1) eingeführt werden.

Bevorzugt sind die Grenzflächen-aktiven Stoffe ausgewählt aus der Gruppe der Additive, wie sie zuvor für den erfindungsgemäßen geschäumten Artikel beschrieben wurden. Auch die verwendeten Mengen dieser Grenzflächen-aktiven Stoffe sind bevorzugt in den Bereichen, wie für den erfindungsgemäßen geschäumten Artikel oben beschrieben.

Bevorzugt sind die Tenside ausgewählt aus der Gruppe der Additive, wie sie zuvor für den erfindungsgemäßen geschäumten Artikel beschrieben wurden. Auch die verwendeten Mengen dieser Tenside sind bevorzugt in den Bereichen, wie für den erfindungsgemäßen geschäumten Artikel oben beschrieben.

Bevorzugt sind die Verdicker ausgewählt aus der Gruppe der Additive, wie sie zuvor für den erfindungsgemäßen geschäumten Artikel beschrieben wurden. Auch die verwendeten Mengen dieser Verdicker sind bevorzugt in den Bereichen, wie für den erfindungsgemäßen geschäumten Artikel oben beschrieben.

Nach Durchführung der Schritte (V1) und optional (V2), V3) und/oder (V4) wird die Mischung (M1) in Schritt (V5) zu einem Schaum aufgeschäumt. Dies erfolgt bevorzugt durch Einbringen von Luft in die Mischung (M1). Dies erfolgt typischerweise durch starkes Rühren bzw. Aufschlagens der Mischung (analog Herstellung von Schlagsahne). In Mischaggregaten wird bevorzugt ein Mischigel verwendet und bevorzugt ein Luftdruck in einem Bereich von 3 bis 10 bar, bevorzugt von 4 bis 8 bar angelegt. Der Schaum, der in Schritt (V5) gebildet wird, wird anschließend für einen angemessenen Zeitraum in Schritt (V6) reifen gelassen. Ein angemessener Zeitraum des Reifens ist bevorzugt in einem Bereich von 1 Sekunde bis 60 Minuten, oder bevorzugt in einem Bereich von 5 Sekunden bis 30 Minuten, oder bevorzugt 1 bis 10 Minuten. Das Reifen in Schritt (V6) wird bevorzugt bei Raumtemperatur, bevorzugt bei 23 °C, erfolgen oder bevorzugt bei erhöhter Temperatur, bevorzugt in einem Bereich von 25 bis 50 °C, oder bevorzugt von 30 bis 40 °C stattfinden. Das Reifen in Schritt (V6), bei dem der Schaum seine Endeigenschaften erhält, wird bevorzugt in dem Behälter vorgenommen, in dem auch das Mischen in Schritt (V1) und Aufschäumen in Schritt (V5) stattfindet. Alternativ kann das Reifen in Schritt (V6) auch durch einen längeren Verbindungsschlauch vom Mischer zum Auftragswerk erreicht werden, bevor die Mischung (M1) auf einem Substrat aufgebracht wird. Durch eine derartige Maßnahme kann ein kontinuierlicher Prozess im Mischer und bei der Auftragung gewährleistet werden. Es kann auch eine Mischung aus beiden Prozessschritten (V6) und (V7) erfolgen.

Das Substrat auf das der Schaum optional in Schritt (V7) aufgebracht werden kann, kann jedes Material beinhalten, das der Fachmann als Beschichtungsunterlage eines Schaums aus Schritt (V5) verwenden würde. Bevorzugt ist das Substrat ausgewählt aus der Gruppe bestehend aus einem Papier, beispielsweise einem Trennpapier, einem Band, beispielsweise einem Förderband aus Kautschuk oder einem Polymer, einem Metall, einer Holzoberfläche, einem Vliesstoff, einer Steinoberfläche, einer Polymeroberfläche oder einer Glasoberfläche und einer Kombination aus mindestens zwei hiervon. Bevorzugt wird ein Trennpapier, ein Film oder ein Vliesstoff verwendet.

Nach dem Aufschäumen in Schritt (V5) und ggf. auch nach dem Reifen in Schritt (V6) und/oder dem Aufbringen auf ein Substrat in Schritt (V7), wird der Schaum in Schritt (V8) getrocknet. Das Trocknen in Schritt (V8) findet bevorzugt auf dem Substrat aus Schritt (V7) statt. Für einen besonders guten Trocknungsvorgang wird der Schaum auf das Substrat mit einer Metall-Rakel mit einer Schichtdicke von 100 µm bis 10 mm, bevorzugt 300 µm bis 7 mm, besonders bevorzugt 1 bis 5 mm gegeben. Der Trocknungsvorgang kann bevorzugt bei erhöhter Temperatur, bevorzugt in einem Bereich von 50 bis 130 °C, oder bevorzugt von 60 bis 100 °C stattfinden. Typischerweise wird an einer Beschichtungsanlage mit verschiedenen Ofenzonen eine ansteigende Temperaturrampe verwendet. Der Schaum wird bevorzugt für einen Zeitraum von 1 bis 60 Minuten, oder für einen Zeitraum von 10 bis 50 Minuten, oder bevorzugt in einem Zeitraum von 3 bis 10 Minuten getrocknet. Um den Trocknungsvorgang zu beschleunigen kann beispielsweise auf die oben angeführte Temperatur erwärmte Luft über den Schaum geleitet oder ein Mikrowellenofen oder ein Infrarotstrahler verwendet werden. Der Schaum weist bevorzugt nach dem Schritt (V8) einen Wassergehalt von 0 bis 3 Gew.-%, oder bevorzugt von 0,2 bis 2 Gew.-%, auf. Der so getrocknete Schaum stellt den erfindungsgemäßen geschäumten Artikel dar.

Ein weiterer Gegenstand der Erfindung ist eine Verwendung des erfindungsgemäßen, geschäumten Artikels bzw. eines nach dem erfindungsgemäßen Verfahren hergestellten geschäumten Artikels als Wundauflage, als Hygieneartikel oder als wearable patch.

Ein weiterer Gegenstand der Erfindung ist ein Kit of parts, mindestens beinhaltend eine Polyurethan-Dispersion (A) wie vorstehend beschrieben und eine Polyurethan-Dispersion (B) wie vorstehend beschrieben, wobei die Polyurethan-Dispersion (A) oder die Polyurethan-Dispersion (B) ein Zusatzmaterial (C) aufweist, wobei die das Zusatzmaterial (C) aufweisende Dispersion (A) oder (B) eine Zell-Viabilität von ≥ 70%, bevorzugt von ≥ 75%, oder bevorzugt von ≥ 80%, in einem Zytotoxtest nach DIN ISO 10993-5:2009-10 aufweist.

### Messmethoden

### Dickenmessung:

Die Schichtdickenbestimmung wurde mit einem Drucklufttaster und zur Ausgabe der Schichtdicke angeschlossenem Display der Firma Heidehain (MT25P) ermittelt.

### Dichtemessung:

Zur Dichtebestimmung wurde ein Probenstück mit Hilfe eines Stanzeisens in der Dimension 5 × 5 cm² (mit abgerundeten Ecken, wobei die Ecken einen Kurvenradius von 3 mm aufweisen) ausgestanzt. Die Höhe wurde aus dem Mittelwert einer 5fach-Bestimmung mittels oben beschriebener Mess-Methode zur Dickenmessung ermittelt. Zur anschließenden Berechnung der Dichte wurde die Masse des Probenstückes an einer Mettler Toledo XS603S Waage bestimmt und zusammen mit dem ermittelten Volumen des Probenstücks konnte die Dichte [g/l] des Probenstücks ermittelt werden.

### Rissfläche:

Zur Bestimmung von Rissflächen in einem Schaum wurde die Oberfläche eines Schaumstücks mit den Maßen von ca. 100 cm² mit einem Flachbettscanner (Epson Perfection V370) eingescannt. Bei sehr hellen Schäumen wurde der Scan bei geöffnetem Deckel durchgeführt, um die Kontrastunterschiede deutlicher hervorzuheben. Bei dunklen Schäumen wird mit geschlossenem Deckel gearbeitet, um einen weißen Hintergrund als Kontrast zu bilden. Damit die Risse beim Scan ausreichend ausgeleuchtet wurden, lagen die Dicken des Schäume in einem Bereich von 0,1 bis 5 mm. Anschließend wurde der Scan mittels der Software ImageJ (Standard Binarisierung -> Image - Adjust - Threshold - Default (0, 172)) binarisiert, was bedeutet, dass oberhalb eines Grauwertschwellenwertes, der bei einem Grauwert von höher als 0,172 gewählt wurde, die Fläche als Teil eines Risses bewertet wurde und unterhalb von 0,172 als glatte Fläche eingestuft wurde. Um zu verhindern, dass auch kleinere Poren als Riss detektiert wurden, wurde der Schwellenwert für die Grundfläche für einen Riss auf > 0,4 mm² festgelegt. Zur Berechnung der prozentualen Rissfläche wurde die detektierte Rissfläche ins Verhältnis zur Gesamtfläche gesetzt.

### Rissbreite:

Zur Bestimmung der Rissfläche eines Schaums, wurde die Oberfläche des Schaumstücks mit einer Fläche von ca. 100 cm² mit einem Flachbettscanner (Epson Perfection V370) eingescannt. Anschließend wurden Bereiche mit Rissen anhand des Grauwertschwellenwertes, wie oben beschrieben, festgelegt und entlang der Risslänge alle 2 mm die Rissbreite mittels der Software ImageJ ermittelt. Anschließend wurde über die gemessenen Werte entlang des Risses der Mittelwert gebildet.

### Risstiefe:

Die Schichtdickenmessung erfolgte wie oben beschrieben mittels Drucklufttaster und einer Spitze deren Durchmesser 3,5 mm beträgt. Bei Rissen, die eine Breite von weniger als 3,5 mm hatten, wurde eine Seite des Risses vor der Messung abgeschnitten, sodass eine Flanke für die Messung der Risstiefe mit dem Drucklufttaster erreichbar war.

### Porengröße:

Die Bestimmung der mittleren Porengröße im Schaumquerschnitt erfolgte in Anlehnung an den wissenschaftlichen Artikel "Methods for Cell Structure Analysis of Polyurethane Foams" (Polyurethanes Expo; 2005; Seite 453 - 465; Landers, R., Venzmer J., Boinowitz, T). Folgende Änderungen wurden hierzu zur Bestimmung der Porengröße vorgenommen: Scannertyp: Epson Perfection V39, Auflösung: 9600dpi, 8bit Graustufen, Edding 390 schwarz, Software: ImageJ Version 1.51n Plugin MorpholibJ.

### Beispiel 1: Herstellung der Polyurethandispersion (A)

1077,2 g PolyTHF^{®} 2000, 409,7 g PolyTHF^{®} 1000, 830,9 g Desmophen^{®} C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:

| | |
|---|---|
| Feststoffgehalt: | 61,6 % |
| Partikelgröße (LKS): | 528 nm |
| pH (23°C): | 7,5 |

### Beispiel 2: Herstellung der Polyurethandispersion (B)

75 g PolyTHF^{®} 1000 und 350 g PolyTHF^{®} 2000 wurden auf 70°C aufgeheizt. Anschließend wurde ein Gemisch aus 33,9 g Hexamethylendiisocyanat , 49,7 g Isophorondiisocyanat, sowie 8,7 g Desmodur N 3300 (HDI-Trimerisat mit einem NCO-Gehalt von ca. 21,8% nach DIN EN ISO 11 909) zugegeben und solange bei 100-115°C gerührt bis der theoretische NCO-Wert unterschritten war. Das fertige Präpolymer wurde mit 920 g Aceton bei 50°C gelöst und anschließend eine Lösung aus 3,2 g Ethylendiamin, 12,9 g Diaminosulfonat, 11,7 g Diethanolamin und 145 g Wasser zudosiert. Die Nachrührzeit betrug 15 min. Danach wurde durch Zugabe von 1080 g Wasser dispergiert. Es folgte die Entfernung des Lösemittels durch Destillation im Vakuum und es wurde eine lagerstabile Dispersion erhalten; der Festgehalt wurde durch Zusatz von Wasser eingestellt.

| | |
|---|---|
| Feststoffgehalt: | 52 % |
| Partikelgröße (LKS): | 307 nm |
| Viskosität: | 105 mPa s |
| Tg Polyurethanharnstoff: | -78,0 °C |

### Thermoplastischer Schaum hergestellt aus zwei speziellen Polyurethandispersionen (Beispiele)

Alle hier beschriebenen Schäume sind weiß und beinhalten keine Farbstoffe oder Pigmente.

### Herstellung des Polyurethanschaums (Vergleichsbeispiel 1):

2 kg der oben beschriebenen wässrigen Polyurethandispersion (A) wurden mit 0,155 kg einer 40 Gew.-% wässrigen Pluronic PE6800-Lösung (der Firma BASF SE, Deutschland), beinhaltend 3,45 Gew.-% Zitronensäure (der Firma Bernd Kraft, Deutschland) mittels eines Dispermaten der Firma VMA-Getzmann GmbH vermischt. Die Mischung wurde anschließend in einem Pico-Mix der Firma Hansa-Mixer zu einem Schaum mit einer Dichte von 200 g/l aufgeschlagen. Der aufgeschlagene Schaum wurde auf ein 30 cm breites polyolefin-beschichtetes Trennpaper (Felix Schoeller Y05200) bei einer Rakeleinstellung von 3000µm aufgegossen. Dieser Schaum wurde bei 120°C °C für 30 Minuten getrocknet.

**Tabelle 1: Schaumeigenschaften des Vergleichsbeispiels 1**

| **Parameter:** | | **Einheit** | **Ergebnis:** |
|---|---|---|---|
| Dicke | | mm | 1,1 |
| Schaumdichte (trocken) | | g/L | 222 |
| Rissfläche | | % | 5,3 |
| Rissbreite | Mittelwert | mm | 2,46 |
| | Std.-Abw. | mm | 1,44 |
| | Kleinster Wert | mm | 0,54 |
| | Größter Wert | mm | 7,97 |
| Risstiefe | Mittelwert | mm | 0,39 |
| | Std.-Abw. | mm | 0,12 |
| Viabilität | | % | 75 |
| Bruchspannung | | kPa | 1095 |
| Bruchdehnung | | % | 239 |
| Mittlere Porengröße | | µm | 427 |

### Herstellung des Polyurethanschaums (Vergleichsbeispiel 2):

4,5 kg der oben beschriebenen wässrigen Polyurethandispersion (A) wurden mit 0,349 kg einer 40 Gew.-% wässrigen Pluronic PE6800-Lösung (der Firma BASF SE, Deutschland) beinhaltend 3,45 Gew.-% Zitronensäure (der Firma Bernd Kraft, Deutschland) und 0,225 kg Niax L6889 (der Firma Momentive Performance Materials Inc., USA) mittels eines Dispermaten der Firma VMA-Getzmann GmbH vermischt. Die Mischung wurde anschließend in einem Pico-Mix der Firma Hansa-Mixer zu einem Schaum mit einer Dichte von 200 g/l aufgeschlagen. Der aufgeschlagene Schaum wurde auf ein 30 cm breites polyolefin-beschichtetes Trennpaper (Felix Schoeller Y05200) bei einer Rakeleinstellung von 3000µm aufgegossen. Der Schaum wurde bei 130 bis 140 °C für 7 Minuten getrocknet.

**Tabelle 2: Schaumeigenschaften des Vergleichsbeispiels 2**

| **Parameter:** | | **Einheit** | **Ergebnis:** |
|---|---|---|---|
| Dicke | | mm | 2,4 |
| Schaumdichte (trocken) | | g/L | 128 |
| Rissfläche | | % | - |
| Rissbreite | Mittelwert | mm | - |
| | Std.-Abw. | mm | - |
| | Kleinster Wert | mm | - |
| | Größter Wert | mm | - |
| Risstiefe | Mittelwert | mm | - |
| | Std.-Abw. | mm | - |
| Viabilität | | % | 18 |
| Bruchspannung | | kPa | 334 |
| Bruchdehnung | | % | 208 |
| Mittlere Porengröße | | µm | 431 |

### Herstellung des Polyurethanschaums (erfindungsgemäßes Beispiel 1):

3,19 kg der oben beschriebenen wässrigen Polyurethandispersion (A) wurden mit 1,367 kg Polyurethandispersion (B) 0,91 kg einer 10 Gew.-% wässrigen Rheolate 208-Dispersion (der Firma Elementis Specialties, Deutschland) und 0,353 kg einer 40 Gew.-% wässrigen Pluronic PE6800-Lösung (der Firma BASF SE, Deutschland) beinhaltend 3,45 Gew.-% Zitronensäure (der Firma Bernd Kraft, Deutschland) mittels eines Dispermaten der Firma VMA-Getzmann GmbH vermischt. Die Mischung wurde anschließend in einem Pico-Mix der Firma Hansa-Mixer zu einem Schaum mit einer Dichte von 200 g/l aufgeschlagen. Der aufgeschlagene Schaum wurde auf ein 30 cm breites polyolefin-beschichtetes Trennpaper (Felix Schoeller Y05200) bei einer Rakeleinstellung von 3000 µm aufgegossen. Anschließend wurde der Schaum bei 130 bis 140 °C für 7 Minuten getrocknet.

**Tabelle 3: Schaumeigenschaften des erfindungsgemäßen Beispiels 1**

| **Parameter:** | | **Einheit** | **Ergebnis:** |
|---|---|---|---|
| Dicke | | mm | 1,8 |
| Schaumdichte (trocken) | | g/L | 159 |
| Rissfläche | | % | 0,0 |
| Rissbreite | Mittelwert | mm | - |
| | Std.-Abw. | mm | - |
| | Kleinster Wert | mm | - |
| | Größter Wert | mm | - |
| Risstiefe | Mittelwert | mm | - |

| Std.-Abw. | | mm | - |
|---|---|---|---|
| | | | |
| Viabilität | | % | 85 |
| Bruchspannung | | kPa | 377 |
| Bruchdehnung | | % | 248 |
| Mittlere Porengröße | | µm | 467 |

In dem Schaum, hergestellt gemäß erfindungsgemäßem Beispiel 1, wurden keine detektierbaren Risse ermittelt, weshalb in der Tabelle 3 keine Angaben zu Rissbreite und Risstiefe zu finden sind. Weiterhin ist beim Vergleich der Ergebnisse aus den Tabellen 1 bis 3 zu erkennen, dass durch die erfindungsgemäße Mischung aus den Dispersionen (A) und (B) ein erfindungsgemäß geschäumter Artikel erhalten wird, der keine Risse und dabei dennoch eine hohe Viabilität aufweist. Die geschäumten Artikel gemäß der Vergleichsbeispiele weisen entweder eine niedrige Viabilität auf oder Risse.

## Patentansprüche

1. Ein geschäumter Artikel, hergestellt durch Mischen einer ersten Polyurethan-Dispersion (A) mit wenigstens einer zweiten Polyurethan-Dispersion (B), gegebenenfalls unter Zusatz weiterer Additive Aufschäumen und anschließendem Trocknen der Mischung, wobei die Polyurethan-Dispersion (A) erhältlich ist, indem
A) isocyanatfunktionelle Prepolymere aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und gegebenenfalls nichtionischen Hydrophilierungsmitteln,
hergestellt, und
B) deren freie NCO-Gruppen dann ganz oder teilweise
B1) gegebenenfalls mit aminofunktionellen Verbindungen mit Molekulargewichten von 32 bis 400 g/mol und
B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, und
wobei die zweite Polyurethan-Dispersion (B) erhältlich ist durch reaktive Umsetzung wenigstens folgender Komponenten:
A. einer aliphatischen Polyisocyanat-Komponente mit einer mittleren Isocyanatfunktionalität von ≥ 1,8 und ≤ 2,6,
B. einer polymeren Polyether-Polyol-Komponente;
C. einer aminofunktionellen Kettenverlängerer-Komponente mit mindestens 2 isocyanatreaktiven Aminogruppen, enthaltend wenigstens eine aminofunktionelle Verbindung C 1., die keine ionischen oder ionogenen Gruppen aufweist und/oder eine aminofunktionelle Verbindung C2., die ionische oder ionogene Gruppen aufweist,
D. gegebenenfalls weiteren hydrophilierenden Komponenten, die unterschiedlich sind von C2.,
E. gegebenenfalls hydroxyfunktionellen Verbindungen mit einem Molekulargewicht von 62 bis 399 mol/g,
F. gegebenenfalls weiteren polymeren Polyolen, welche unterschiedlich sind von B.,
G. einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
H. optional einer aliphatischen Polyisocyanat-Komponente mit einer mittleren Isocyanatfunktionalität von > 2,6 und ≤ 4,
wobei die Komponenten B. und F. zusammen ≤ 30 Gew.-% an Komponente F., bezogen auf die Gesamtmasse der Komponenten B. und F. enthalten.

2. Der geschäumte Artikel gemäß Anspruch 1, wobei der geschäumte Artikel mindestens eine, bevorzugt mindestens zwei, oder bevorzugt alle der folgenden Eigenschaften i. bis xiii. aufweist:
i. der geschäumte Artikel keine Risse aufweist, die eine Breite von ≥ 2 mm, bevorzugt von ≥ 1 mm, mehr bevorzugt von ≥ 0,5 mm, besonders bevorzugt von ≥ 0,1 mm aufweisen;
ii. der geschäumte Artikel auf seiner Oberfläche keine Risse aufweist, die tiefer als 0,4 mm, bevorzugt tiefer als 0,2 mm oder bevorzugt tiefer als 0,1mm sind;
iii. der geschäumte Artikel eine Rissfläche von ≤ 6%, bevorzugt ≤ 3% oder bevorzugt ≤ 0,5% aufweist, bezogen auf die Gesamtoberfläche des geschäumten Artikels;
iv. der geschäumte Artikel eine Viabilität der Zellen von ≥ 70% bzw. eine Klassifizierung von 0 bis 2 in einem Zytotoxtest nach DIN ISO 10993-5:2009-10 aufweist;
v. der geschäumte Artikel eine Dichte in einem Bereich von 80 bis 500 g/L, bevorzugt 100 bis 400 g/L, oder bevorzugt 120 bis 300 g/L aufweist;
vi. der geschäumte Artikel eine Dicke (D) in einem Bereich von 0,1 bis 100 mm, bevorzugt in einem Bereich von 0,5 bis 50 mm, bevorzugt in einem Bereich von 1 bis 10 mm aufweist;
vii. der geschäumte Artikel eine Bruchspannung in einem Bereich von 100 bis 1100 kPa, bevorzugt in einem Bereich von 200 bis 500 kPa, nach DIN EN ISO 527-2:2012-06 gemessen, aufweist;
viii. der geschäumte Artikel eine Bruchdehnung in einem Bereich von 100 bis 500 %, bevorzugt in einem Bereich von 150 bis 400 %, nach DIN EN ISO 527-2:2012-06 gemessen, aufweist;
ix. dass der Polyurethanharnstoff gebildet aus der Polyurethandispersion (B) amorph ist und einen Tg ≤ - 25 °C, bevorzugt ≤ - 40 °C, oder bevorzugt ≤ - 50 °C, oder bevorzugt ≤ - 70 °C aufweist, bestimmt mittels dynamischer Differenzkalorimetrie in Anlehnung an die DIN EN 61006, Verfahren A;
x. der geschäumte Artikel einen Schmelz- oder Erweichungsbereich von ≤ 180°C, bevorzugt ≤ 150°C bei einem maximalen Druck von 4 bar hat und in diesem Bereich vollständig thermoplastisch verarbeitbar ist;
xi. der geschäumte Artikel eine mittlere Porengröße in einem Bereich von 200 bis 750 µm, bevorzugt in einem Bereich von 300 bis 600 µm aufweist;
xii. der geschäumte Artikel eine Deckschicht, bevorzugt ein thermoplastisches Polyurethan oder ein thermoplastisches Polyethylen enthaltend, mindestens auf einer Oberfläche des geschäumten Artikels aufweist;
xiii. dass der geschäumte Artikel Bestandteil eines Verbundmaterials ist.

3. Der geschäumte Artikel gemäß Anspruch 1 oder 2, wobei das Gewichts-Verhältnis der Polyurethan-Dispersion (A) zu der Polyurethan-Dispersion (B) in einem Bereich von 1:1 bis 5:1, bezogen auf die Gesamtmasse aus den Massen der Dispersionen (A) und (B), liegt.

4. Der geschäumte Artikel gemäß einem der vorhergehenden Ansprüche, wobei ein aus der Dispersion (B) gebildeter Film eine Bruchspannung von ≤ 5 MPa, bevorzugt ≤ 3,5 MPa oder bevorzugt ≤ 2,5 MPa bei gleichzeitiger Bruchdehnung von ≥ 1750 %, bevorzugt ≥ 2000 % auf.

5. Der geschäumte Artikel gemäß einem der vorhergehenden Ansprüche, wobei als Hydrophilierungsmittel B2) Sulfongruppen-haltige Hydrophilierungsmitteln verwendet werden.

6. Der geschäumte Artikel gemäß einem der vorhergehenden Ansprüche, wobei die Dispersion (A) einen Feststoffgehalt an Polyurethan von 52 bis 65 Gew.-%, bevorzugt von 55 bis 63 Gew.-%, oder bevorzugt von 57 bis 62 Gew.-%, bezogen auf die Gesamtmasse der Dispersion (A) aufweist.

7. Der geschäumte Artikel gemäß einem der vorhergehenden Ansprüche, wobei die Komponente A. oder A1) Isophorondiisocyanat und/oder Hexamethylendiisocyanat ist.

8. Der geschäumte Artikel gemäß einem der vorhergehenden Ansprüche, wobei die Komponente B. Poly(tetramethylenglykol)polyetherpolyole enthält oder daraus besteht.

9. Der geschäumte Artikel gemäß einem der vorhergehenden Ansprüche, wobei die Komponente B. ein Gemisch aus mindestens zwei Poly(tetramethylenglykol)polyetherpolyolen enthält oder daraus besteht, wobei sich die mindestens zwei Poly(tetramethylenglykol)polyetherpolyole in ihren zahlenmittleren Molekulargewichten unterscheiden.

10. Der geschäumte Artikel gemäß einem der vorhergehenden Ansprüche, **dadurch gekennzeichnet, dass** es sich bei der Komponente D. um nichtionisch hydrophilierende Komponenten handelt.

11. Der geschäumte Artikel gemäß einem der vorhergehenden Ansprüche, **dadurch gekennzeichnet, dass** die Komponente H. eingesetzt wird und das molare Verhältnis von Komponente G. zu Komponente H. 5:1 bis 1:5 beträgt.

12. Der geschäumte Artikel gemäß einem der vorhergehenden Ansprüche, **dadurch gekennzeichnet, dass** die Polyurethandispersion (B) erhältlich ist indem isocyanatfunktionelle Polyurethanpräpolymere a) aus den Komponenten A., B. und gegebenenfalls D. und/oder C2., sowie gegebenenfalls den Verbindungen E. und/oder H. hergestellt werden und deren freie NCO-Gruppen anschließend ganz oder teilweise mit der aminofunktionellen Kettenverlängerer-Komponente C., sowie der Komponente G. und gegebenenfalls der Komponente D. und H. umgesetzt werden.

13. Ein Verfahren zur Herstellung eines geschäumten Artikels gemäß Anspruch 1, wobei das Verfahren mindestens die folgenden Schritte enthält:
(V1) Mischen der Polyurethandispersion (A) und der Polyurethandispersion (B) unter Erhalt einer Mischung (M1),
(V2) ggf. Zugabe zu (M1) mindestens eines Grenzflächen aktiven Stoffes, wie ein Schaumstabilisator, der bevorzugt nicht-zytotoxisch ist,
(V3) ggf. Zugabe zu (M1) mindestens eines Tensid, das bevorzugt nicht-zytotoxisch ist,
(V4) ggf. Zugabe zu (M1) eines Verdickers, bevorzugt eines assoziativen PU-Verdickers, der bevorzugt nicht-zytotoxisch ist,
(V5) Aufschäumen der Mischung (M1) zu einem Schaum, bevorzugt durch Einbringen von Luft in die Mischung (M1)
(V6) ggf. Reifen des Schaums aus Schritt (V2),
(V7) ggf. Aufbringen des in Schritt (V2) oder (V3) gebildeten Schaums auf ein Substrat,
(V8) Trocknen des Schaums unter Bildung des geschäumten Artikels.

14. Eine Verwendung des geschäumten Artikels gemäß einem der Ansprüche 1 bis 12 oder hergestellt gemäß Anspruch 13 als Wundauflage, einem Hygieneartikel oder einem wearable patch.

15. Ein Kit of parts, mindestens beinhaltend eine Polyurethan-Dispersion (A) wie in Anspruch 1 beschrieben und eine Polyurethan-Dispersion (B) wie in Anspruch 1 beschrieben, wobei die Polyurethan-Dispersion (A) oder die Polyurethan-Dispersion (B) ein Zusatzmaterial (C) aufweist, wobei die das Zusatzmaterial (C) aufweisende Dispersion (A) oder (B) eine Zell-Viabilität von ≥ 70 % in einem Zytotoxtest oder eine Klassifikation von 0 bis 2 nach DIN ISO 10993-5:2009-10 aufweist.

## Claims

1. Foamed article produced by mixing a first polyurethane dispersion (A) with at least one second polyurethane dispersion (B), optionally with addition of further additives, foaming and subsequently drying the mixture, wherein the polyurethane dispersion (A) is obtainable by preparing
A) isocyanate-functional prepolymers from
A1) organic polyisocyanates
A2) polymeric polyols having number-average molecular weights of 400 to 8000 g/mol and OH functionalities of 1.5 to 6 and
A3) optionally hydroxyl-functional compounds having molecular weights of 62 to 399 g/mol and
A4) optionally isocyanate-reactive, anionic or potentially anionic and optionally nonionic hydrophilizing agents,
and
B) the free NCO groups thereof are then wholly or partly reacted
B1) optionally with amino-functional compounds having molecular weights of 32 to 400 g/mol and
B2) with amino-functional, anionic or potentially anionic hydrophilizing agents
by chain extension and the prepolymers are dispersed in water before, during or after step B), and
wherein the second polyurethane dispersion (B) is obtainable by reactive conversion of at least the following components:
A. an aliphatic polyisocyanate component having an average isocyanate functionality of ≥ 1.8 and ≤ 2.6,
B. a polymeric polyether polyol component;
C. an amino-functional chain extender component having at least 2 isocyanate-reactive amino groups, comprising at least one amino-functional compound C1. that does not have any ionic or ionogenic groups and/or an amino-functional compound C2. that has ionic or ionogenic groups,
D. optionally further hydrophilizing components other than C2.,
E. optionally hydroxyl-functional compounds having a molecular weight of 62 to 399 mol/g,
F. optionally further polymeric polyols other than B. ,
G. a compound having exactly one isocyanate-reactive group or a compound having more than one isocyanate-reactive group where only one of the isocyanate-reactive groups reacts with the isocyanate groups present in the reaction mixture under the reaction conditions chosen, and
H. optionally an aliphatic polyisocyanate component having an average isocyanate functionality of > 2.6 and ≤ 4,
where components B. and F. together contain ≤ 30% by weight of component F., based on the total mass of components B. and F..

2. Foamed article according to Claim 1, wherein the foamed article has at least one, preferably at least two, or preferably all, of the following properties i. to xiii.:
i. the foamed article has no cracks having a width of ≥ 2 mm, preferably of ≥ 1 mm, more preferably of ≥ 0.5 mm, especially preferably of ≥ 0.1 mm;
ii. the foamed article has no surface cracks deeper than 0.4 mm, preferably deeper than 0.2 mm or preferably deeper than 0.1 mm;
iii. the foamed article has a crack area of ≤ 6%, preferably ≤ 3% or preferably ≤ 0.5%, based on the total area of the foamed article;
iv. the foamed article has a cell viability of ≥ 70% or a classification of 0 to 2 in a cytotoxicity test to DIN ISO 10993-5:2009-10;
v. the foamed article has a density within a range from 80 to 500 g/l, preferably 100 to 400 g/l, or preferably 120 to 300 g/l;
vi. the foamed article has a thickness (D) within a range from 0.1 to 100 mm, preferably within a range from 0.5 to 50 mm, preferably within a range from 1 to 10 mm;
vii. the foamed article has a tensile stress at break within a range from 100 to 1100 kPa, preferably within a range from 200 to 500 kPa, measured to DIN EN ISO 527-2:2012-06;
viii. the foamed article has an elongation at break within a range from 100% to 500%, preferably within a range from 150% to 400%, measured to DIN EN ISO 527-2: 2012-06;
ix. the polyurethaneurea formed from the polyurethane dispersion (B) is amorphous and has a Tg ≤ - 25°C, preferably ≤ -40°C, or preferably ≤ -50°C, or preferably ≤ -70°C, determined by means of differential scanning calorimetry in accordance with DIN EN 61006, Method A;
x. the foamed article has a melting or softening range of ≤ 180°C, preferably ≤ 150°C, at a maximum pressure of 4 bar and has full thermoplastic processibility within this range;
xi. the foamed article has an average pore size within a range from 200 to 750 pm, preferably within a range from 300 to 600 µm;
xii. the foamed article has an outer layer, preferably containing a thermoplastic polyurethane or a thermoplastic polyethylene, at least on one surface of the foamed article;
xiii. the foamed article is part of a composite material.

3. Foamed article according to Claim 1 or 2, wherein the weight ratio of the polyurethane dispersion (A) to the polyurethane dispersion (B) is within a range from 1:1 to 5:1, based on the total mass of the masses of dispersions (A) and (B).

4. Foamed article according to any of the preceding claims, wherein a film formed from the dispersion (B) has a tensile strain at break of ≤ 5 MPa, preferably ≤ 3.5 MPa or preferably ≤ 2.5 MPa, combined with an elongation at break of ≥ 1750%, preferably ≥ 2000%.

5. Foamed article according to any of the preceding claims, wherein the hydrophilizing agents B2) used are hydrophilizing agents containing sulfone groups.

6. Foamed article according to any of the preceding claims, wherein the dispersion (A) has a solids content of polyurethane of 52% to 65% by weight, preferably 55% to 63% by weight, or preferably 57% to 62% by weight, based on the total mass of the dispersion (A).

7. Foamed article according to any of the preceding claims, wherein component A. or A1) is isophorone diisocyanate and/or hexamethylene diisocyanate.

8. Foamed article according to any of the preceding claims, wherein component B. comprises or consists of poly(tetramethylene glycol) polyether polyols.

9. Foamed article according to any of the preceding claims, wherein component B. comprises or consists of a mixture of at least two poly(tetramethylene glycol) polyether polyols, wherein the at least two poly(tetramethylene glycol) polyether polyols are of different number-average molecular weight.

10. Foamed article according to any of the preceding claims, **characterized in that** component D. comprises nonionically hydrophilizing components.

11. Foamed article according to any of the preceding claims, **characterized in that** component H. is used and the molar ratio of component G. to component H. is 5:1 to 1:5.

12. Foamed article according to any of the preceding claims, **characterized in that** the polyurethane dispersion (B) is obtainable by preparing isocyanate-functional polyurethane prepolymers a) from components A., B. and optionally D. and/or C2., and optionally compounds E. and/or H., and the free NCO groups thereof are then wholly or partly reacted with the amino-functional chain-extender component C., and also component G. and optionally components D. and H..

13. Process for producing a foamed article according to Claim 1, wherein the process comprises at least the following steps:
(V1) mixing the polyurethane dispersion (A) and the polyurethane dispersion (B) to obtain a mixture (M1),
(V2) optionally adding to (M1) at least one interface-active substance, such as a foam stabilizer, that is preferably non-cytotoxic,
(V3) optionally adding to (M1) at least one surfactant that is preferably non-cytotoxic,
(V4) optionally adding to (M1) a thickener, preferably an associative PU thickener, that is preferably non-cytotoxic,
(V5) foaming the mixture (M1) to give a foam, preferably by introducing air into the mixture (M1),
(V6) optionally maturing the foam from step (V2),
(V7) optionally applying the foam formed in step (V2) or (V3) to a substrate,
(V8) drying the foam to form the foamed article.

14. Use of the foamed article according to any of Claims 1 to 12 or produced according to Claim 13 as wound dressing, a hygiene article or a wearable patch.

15. Kit of parts at least including a polyurethane dispersion (A) as described in Claim 1 and a polyurethane dispersion (B) as described in Claim 1, wherein the polyurethane dispersion (A) or the polyurethane dispersion (B) includes an added material (C), wherein the dispersion (A) or (B) including the added material (C) has a cell viability of ≥ 70% in a cytotoxicity test or a classification of 0 to 2 according to DIN ISO 10993-5:2009-10.

## Revendications

1. Article expansé, fabriqué par mélange d'une première dispersion de polyuréthanes (A) à au moins une deuxième dispersion de polyuréthanes (B), éventuellement avec addition d'autres additifs, expansion puis séchage du mélange, la dispersion de polyuréthanes (A) pouvant être obtenue par
A) fabrication de prépolymères à fonctionnalité isocyanate, à partir
A1) de polyisocyanates organiques
A2) de polyols polymères ayant une masse moléculaire moyenne en nombre de 400 à 8 000 g/mol et une fonctionnalité OH de 1,5 à 6, et
A3) éventuellement de composés à fonctionnalité hydroxy, ayant une masse moléculaire de 62 à 399 g/mol et
A4) éventuellement d'agents d'hydrophilisation à activité isocyanate, anioniques ou potentiellement anioniques et éventuellement non ioniques, et
B) puis par réaction complète ou partielle, avec extension de chaîne, de leurs groupes NCO libres,
B1) éventuellement avec des composés à fonctionnalité amino ayant une masse moléculaire de 32 à 400 g/mol et
B2) d'agents d'hydrophilisation à fonctionnalité amino, anioniques ou potentiellement anioniques,
et dispersion des polymères dans l'eau avant, pendant ou après l'étape B), et
la deuxième dispersion de polyuréthanes (B) pouvant être obtenue par réaction réactive d'au moins les composants suivants :
A. un composant polyisocyanate aliphatique ayant une fonctionnalité isocyanate moyenne de ≥ 1,8 à ≤ 2,6,
B. un composant polyéther-polyol polymère ;
C. un composant extendeur de chaîne à fonctionnalité amino ayant au moins 2 groupes amino réactifs vis-à-vis des isocyanates, contenant au moins un composé à fonctionnalité amino C1., qui ne comprend aucun groupe ionique ou ionogène, et/ou comprend un composé à fonctionnalité amino C2., qui comprend des groupes ioniques ou ionogènes,
D. éventuellement d'autres composants d'hydrophilisation qui sont différents de C2.,
E. éventuellement des composés à fonctionnalité hydroxy ayant une masse moléculaire de 62 à 399 mol/g,
F. éventuellement d'autres polyols polymères différents de B.,
G. un composé qui comprend exactement un groupe réactif vis-à-vis des isocyanates, ou un composé qui comprend plus d'un groupe réactif vis-à-vis des isocyanates, un seul des groupes réactifs vis-à-vis des isocyanates réagissant, dans les conditions de réaction choisies, avec les groupes isocyanates présents dans le mélange réactionnel et
H. en option un composant polyisocyanate aliphatique ayant une fonctionnalité isocyanate moyenne de > 2,6 à ≤ 4,
les composants B. et F. contenant ensemble ≤ 30 % en poids du composant F., par rapport au poids total des composants B. et F..

2. Article expansé selon la revendication 1, l'article expansé présentant au moins l'une, de préférence deux ou de préférence la totalité des propriétés i. à xiii. ci-après :
i. l'article expansé ne présente aucune fissure ayant une largeur ≥ 2mm, de préférence ≥ 1 mm, plus particulièrement ≥ 0,5 mm, tout particulièrement ≥ 0,1 mm ;
ii. l'article expansé ne présente sur sa surface aucune fissure plus profonde que 0,4 mm, de préférence plus profonde que 0,2 mm ou de préférence plus profonde que 0,1 mm ;
iii. l'article expansé présente une aire des fissures ≤ 6 %, de préférence ≤ 3 % ou de préférence ≤ 0,5 %, par rapport à l'aire totale de l'article expansé ;
iv. l'article expansé présente une viabilité des alvéoles ≥ 70 %, ou une classification de 0 à 2 dans un essai de cytotoxicité selon DIN ISO 10993-5:2009-10 ;
v. l'article expansé présente une masse volumique dans la plage de 80 à 500 g/l, de préférence de 100 à 400 g/l ou de préférence de 120 à 300 g/l ;
vi. l'article expansé présente une épaisseur (D) dans la plage de 0,1 à 100 mm, de préférence dans la plage de 0,5 à 50 mm, de préférence dans la plage de 1 à 10 mm ;
vii. l'article expansé présente une tension de rupture dans la plage de 100 à 1 100 kPa, de préférence dans la plage de 200 à 500 kPa, mesurée selon DIN EN ISO 527-2:2012-06 ;
viii. l'article expansé présente un allongement à la rupture dans la plage de 100 à 500 %, de préférence dans la plage de 150 à 400 %, mesuré selon DIN EN ISO 527-2:2012-06 ;
ix. que la polyuréthane-urée formée à partir de la dispersion de polyuréthanes (B) est amorphe et présente une Tg ≤ -25 °C, de préférence ≤ -40 °C ou de préférence ≤ -50 °C ou de préférence ≤ -70 °C, déterminée par analyse calorimétrique différentielle selon DIN EN 61006, méthode A ;
x. l'article expansé présente une plage de fusion ou de ramollissement ≤ 180 °C, de préférence ≤ 150 °C sous une pression maximale de 4 bar, et peut dans cette plage être entièrement mis en œuvre par voie thermoplastique ;
xi. l'article expansé présente une grosseur moyenne de pore dans la plage de 200 à 750 µm, de préférence dans la plage de 300 à 600 µm ;
xii. l'article expansé présente, au moins sur une surface de l'article expansé, une couche de revêtement, de préférence un polyuréthane thermoplastique ou un polyéthylène thermoplastique ;
xiii. que l'article expansé est un constituant d'un matériau composite.

3. Article expansé selon la revendication 1 ou 2, dans lequel le rapport en poids de la dispersion de polyuréthanes (A) à la dispersion de polyuréthanes (B) se trouve dans la plage de 1:1 à 5:1, par rapport au poids total des dispersions (A) et (B).

4. Article expansé selon l'une des revendications précédentes, dans lequel un film formé à partir de la dispersion (B) présente une tension de rupture ≤ 5 MPa, de préférence ≤ 3,5 MPa ou de préférence ≤ 2,5 MPa, pour un allongement à la rupture simultané ≥ 1 750 %, de préférence ≥ 2 000 %.

5. Article expansé selon l'une des revendications précédentes, les agents d'hydrophilisation B2) utilisés étant des agents d'hydrophilisation contenant des groupes sulfo.

6. Article expansé selon l'une des revendications précédentes, la dispersion (A) présentant une teneur en extrait sec de polyuréthane de 52 à 65 % en poids, de préférence de 55 à 63 % en poids ou de préférence de 57 à 62 % en poids par rapport au poids total de la dispersion (A).

7. Article expansé selon l'une des revendications précédentes, le composant A. ou A1) étant le diisocyanate d'isophorone et/ou le diisocyanate d'hexaméthylène.

8. Article expansé selon l'une des revendications précédentes, le composant B. contenant des poly(tétraméthylèneglycol)polyétherpolyols ou en étant constitué.

9. Article expansé selon l'une des revendications précédentes, le composant B. contenant un mélange d'au moins deux poly(tétraméthylèneglycol)polyétherpolyols ou en étant constitué, les au moins deux poly(tétraméthylèneglycol)polyétherpolyols se distinguant par leur masse moléculaire moyenne en nombre.

10. Article expansé selon l'une des revendications précédentes, **caractérisé en ce qu'**il s'agit, pour le composant D., de composants d'hydrophilisation non ionique.

11. Article expansé selon l'une des revendications précédentes, **caractérisé en ce qu'**on utilise le composant H. et que le rapport en moles du composant G. au composant H. est de 5:1 à 1:5.

12. Article expansé selon l'une des revendications précédentes, **caractérisé en ce que** la dispersion de polyuréthanes (B) peut être obtenue par fabrication de prépolymères de polyuréthane à fonctionnalité isocyanate a) à partir des composants A., B. et éventuellement D. et/ou C2., ainsi qu'éventuellement à partir des composés E. et/ou H., et que l'on fait ensuite réagir en totalité ou en partie leurs groupes NCO libres avec le composant extendeur de chaîne à fonctionnalité amino C., ainsi que le composant G. et éventuellement les composants D. et H..

13. Procédé de fabrication d'un article expansé selon la revendication 1, le procédé comprenant au moins les étapes suivantes :
(V1) mélange de la dispersion de polyuréthanes (A) et de la dispersion de polyuréthanes (B) pour obtenir un mélange (M1),
(V2) éventuellement addition à (M1) d'au moins une substance à activité interfaciale, telle qu'un stabilisant de mousse qui de préférence n'est pas cytotoxique,
(V3) éventuellement addition à (M1) d'au moins un tensioactif qui de préférence n'est pas cytotoxique,
(V4) éventuellement addition à (M1) d'un épaississant, de préférence un épaississant PU associatif, qui de préférence n'est pas cytotoxique,
(V5) expansion du mélange (M1) pour obtenir une mousse, de préférence par introduction d'air dans le mélange (M1)
(V6) éventuellement maturation de la mousse de l'étape (V2),
(V7) éventuellement application, sur un substrat, de la mousse formée dans l'étape (V2) ou (V3),
(V8) séchage de la mousse avec formation de l'article expansé.

14. Utilisation de l'article expansé selon l'une des revendications 1 à 12 ou fabriqué selon la revendication 13 comme pansement, article d'hygiène ou timbre transdermique.

15. Trousse de pièces contenant au moins une dispersion de polyuréthanes (A) telle que décrite dans la revendication 1 et une dispersion de polyuréthanes (B) telle que décrite dans la revendication 1, la dispersion de polyuréthanes (A) ou la dispersion de polyuréthanes (B) comportant un matériau additif (C), la dispersion (A) ou (B) comportant le matériau additif (C) présentant une viabilité des alvéoles ≥ 70 % dans un essai de cytotoxicité ou une classification de 0 à 2 selon DIN ISO 10993-5:2009-10.
